(19) Europäisches Patentamt
European Patent Office
Office européen des brevets

(11) **EP 4 003 416 B1**

(12) **EUROPEAN PATENT SPECIFICATION**

(45) Date of publication and mention of the grant of the patent:
**08.10.2025 Bulletin 2025/41**

(21) Application number: **20746232.6**

(22) Date of filing: **31.07.2020**

(51) International Patent Classification (IPC):
***A61K 39/395*** *(2006.01)* ***C12Q 1/6804*** *(2018.01)*
***C12Q 1/6862*** *(2018.01)*

(52) Cooperative Patent Classification (CPC):
(C-Sets available)
**C12Q 1/6804; C12Q 1/6862** (Cont.)

(86) International application number:
**PCT/EP2020/071688**

(87) International publication number:
**WO 2021/019081 (04.02.2021 Gazette 2021/05)**

(54) **C3/C5 CONVERTASE ASSAYS**

C3/C5-KONVERTASE-ASSAYS

ANALYSES CONVERTASE C3/C5

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2019 EP 19189415**

(43) Date of publication of application:
**01.06.2022 Bulletin 2022/22**

(73) Proprietor: **Universitätsklinikum Hamburg-Eppendorf**
**20246 Hamburg (DE)**

(72) Inventors:
- **WIECH, Thorsten**
  **20255 Hamburg (DE)**
- **ZIPFEL, Peter**
  **07743 Jena (DE)**
- **PERSON, Fermin**
  **14163 Berlin (DE)**
- **WULF, Sonia**
  **22529 Hamburg (DE)**

(74) Representative: **Stüven, Ralf**
**RGTH Patentanwälte PartGmbB**
**Kirchenhang 32b**
**21073 Hamburg (DE)**

(56) References cited:
**WO-A1-2011/123044**
**WO-A1-2014/055835**
**WO-A1-2015/070041**
**WO-A1-2018/081400**
**WO-A1-2019/099950**
**WO-A2-03/068150**
**WO-A2-2004/112572**

- **SODERBERG O ET AL: "Characterizing proteins and their interactions in cells and tissues using the in situ proximity ligation assay", METHODS, ACADEMIC PRESS, NL, vol. 45, no. 3, 1 July 2008 (2008-07-01), pages 227 - 232, XP023437838, ISSN: 1046-2023, [retrieved on 20080711], DOI: 10.1016/J.YMETH.2008.06.014**
- **MATTHEW C. PICKERING ET AL: "C3 glomerulopathy: consensus report", KIDNEY INTERNATIONAL, vol. 84, no. 6, 1 December 2013 (2013-12-01), LONDON, GB, pages 1079 - 1089, XP055646662, ISSN: 0085-2538, DOI: 10.1038/ki.2013.377**
- **ZHENG JING-MIN ET AL: "Lectin-induced renal local complement activation is involved in tubular interstitial injury in diabetic nephropathy", CLINICA CHIMICA ACTA, ELSEVIER BV, AMSTERDAM, NL, vol. 482, 28 March 2018 (2018-03-28), pages 65 - 73, XP085401032, ISSN: 0009-8981, DOI: 10.1016/J.CCA.2018.03.033**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6804, C12Q 2543/101;**
**C12Q 1/6862, C12Q 2543/101**

## Description

### FIELD OF THE INVENTION

The present invention concerns an in vitro method for classifying a disorder associated with the complement system comprising the detection of the presence of C3/C5 convertase complexes. Moreover, the invention refers to an in vitro method for determining whether a patient will be responsive to a modulator of the complements system, an in vitro method for determining the disorder course of a patient with a disorder associated with the complement system and an in vitro method for determining whether a candidate compound is suitable for the treatment of a disorder of the complement system, and the respective kits.

### BACKGROUND OF THE INVENTION

There is growing evidence that deregulated complement activation contributes to or drives the pathogenesis of various diseases. Thus, targeting complement is a therapeutic option (Thurman et al., 2015; Zipfel et al., 2015). C5 convertase inhibition is an established therapy in atypical hemolytic uremic syndrome and several novel drugs target other components of the complement cascade (Reddy et al., 2017, Ricklin et al. 2018). Currently, the diagnosis of complement activation in kidney diseases is primarily based on immunohistochemical detection of deposited complement activation products in tissue, the detection of consumption of complement components in plasma or body fluids and the generation of activation fragments or cleavage products.

The activation of the complement system occurs by three different pathways; the classical/lectin pathway and the alternate pathway. The diseases of the complement system differ in their prognosis and therapy. Classifying, i.e. discriminating the various diseases and subgroups of diseases would be advantageous in order to get more insights into the pathogenesis, provide better diagnosis and more specific and efficient treatment strategies, and thus to achieve better prognosis for the specific diseases. Moreover, assays for testing potential drug candidates for the diseases associated with the deregulated complement system are needed.

Complement activation comprises three different pathways, the classical, the lectin and the alternative pathway.

In the classical pathway and the lectin pathway, the C3 convertase consisting of C4b and C2b cleaves C3 to C3b and C3a. In this classical pathway, downstream thereof, the C5 convertase consisting of C4b, C2b and C3b cleaves C5 to C5b and C5a.

In the alternative pathway, the cleavage of C3 to C3b and C3a is catalyzed by the C3 convertase consisting of C3b and Bb and subsequently the cleavage of C5 to C5b and C5a is promoted by the C5 convertase consisting of two C3b subunits and Bb.

WO 03/068150 describes methods of diagnosing and treating antibody-mediated arthritis, and discloses that an abnormality in the alternative pathway of complement activation is involved in antibody-mediated arthritis.

Up to now, determining whether a disease is caused or aggravated by aberrations in the classical/lectin and/or alternative pathway has been challenging and often impossible. Moreover, a method to identify, localize and differentiate complement convertases directly in tissues and other biological specimen has been lacking.

### OBJECTIVES AND SUMMARY OF THE INVENTION

It is an objective of the invention to establish a method to detect molecular complexes of the complement activation pathway or to discriminate between aberration of the classical/lectin and alternative pathways. More specifically, a method to discriminate assembled C3/C5 convertases of the classical/lectin and alternative pathways is provided. Close proximity of C2b and C4b is used to identify assembled C3/C5 convertase of the classical/lectin pathway and close proximity of C3b and Bb for measuring the C3/C5 convertase of the alternative pathway. This allows the diagnosis, assessment of disease course/activity and/or treatment efficacy of disorders associated with the complement system.

The present invention further provides a method that allows discriminating between the classical pathway, the lectin pathway and the alternative pathway.

In general, the invention relates to an *in vitro* method for classifying a disorder associated with the complement system, comprising the following steps:

(a) Providing a sample of a subject;
(b) Detecting the presence of C3/C5 convertase complex in the sample of the subject comprising the following steps:

  (i) determining the amount of C4b/C2b complexes in the sample and
  (ii) determining the amount of C3b/Bb complexes in the sample,

wherein an increased amount of C4b/C2b complexes compared to a control or predetermined threshold is indicative for a disorder associated with the classical/lectin pathway and wherein an increased amount of C3b/Bb complexes compared to a control or predetermined threshold is indicative for a disorder associated with the alternative pathway;
(c) Classifying the disorder associated with the complement system based on the result of step (b).

The *in vitro* method for classifying a disorder associated with the complement system of the invention comprises the following steps:

(a) providing a sample of a subject;
(b) detecting the presence of C3/C5 convertase complex in the sample of the subject;
(c) classifying the disorder associated with the complement system based on the result of step (b).

Step b) comprises the following steps:

(i) Determining the amount of C4b/C2b complexes in the sample and
(ii) Determining the amount of C3b/Bb complexes in the sample,

wherein an increased amount of C4b/C2b complexes compared to a control or predetermined threshold is indicative for a disorder associated with the classical/lectin pathway and wherein an increased amount of C3b/Bb complexes compared to a control or predetermined threshold is indicative for a disorder associated with the alternative pathway.
In one embodiment, step b) further comprises the following steps:

(i) Determining the amount of Ig/C1q complex in the sample; and/or
(ii) Determining the amount of MASP1/3/MBL complex and/or MASP2/MBL complex in the sample.

In one embodiment, step b) thus comprises the following steps:

(i) Determining the amount of C4b/C2b complexes in the sample; and
(ii) Determining the amount of C3b/Bb complexes in the sample; and
(iii) Determining the amount of Ig/C1q complex in the sample; and/or
(iv) Determining the amount of MASP1/3/MBL complex and/or MASP2/MBL complex in the sample.

Since the C3 convertase and the C5 convertase of the classical and the lectin pathways comprise the complex of C4b and C2b, the determination of the interaction of C4b and C2b allows the specific detection of the C3/C5 convertase of the classical/lectin pathway.
By the accurate measurement of the C3/C5 convertase specific for the classical/lectin pathway, the invention thus provides for the first time an assay that allows to discriminate whether a specific disorder of the complement system is due to increased activity of the classical/lectin pathway, in particular due to the increased number of C3/C5 convertase complex of the classical/lectin pathway or upstream events that lead to an increased number of C3/C5 convertase complexes of the classical/lectin pathway.
The invention thus provides for the first time an assay that allows to identify the complexes in the biological specimen and furthermore to determine whether a specific disorder of the complement system correlates with or is due to increased activity of the classical/lectin pathway, in particular due to the increased number of C3/C5 convertase complex of the classical/lectin pathway or upstream events that lead to an increased number of C3/C5 convertase complexes of the classical/lectin pathway.
Correspondingly, the C3 convertase and the C5 convertase of the alternative pathway comprise the complex of C3b and Bb, the determination of the interaction of C3b and Bb allows the specific detection of the C3/C5 convertase of the alternative pathway.
By the accurate measurement of the C3/C5 convertase specific for the alternative pathway, the invention thus provides for the first time an assay that allows to directly determine whether a specific disease like a disorder of the complement system is due to aberrations of the alternative pathway, in particular due to the increased activity of the C3/C5 convertase complex of the alternative pathway or upstream events that lead to an increased number of C3/C5 convertase complexes of the alternative pathway.
Further, measuring Ig/C1q complex allows determining whether the classical pathway is activated. Measuring MASP1/3/MBL complex and/or MASP2/MBL allows determining whether the lectin pathway is activated. Hence, the provided method also distinguishes between the lectin pathway and the classical pathway.
The invention thus provides for the first time an assay that allows to directly determine whether a specific disease like a disorder of the complement system is due to aberrations of the classical pathway or the lectin pathway.

The invention also provides for the first time an assay that allows to directly determine whether a specific disease like a disorder of the complement system is due to aberrations of the classical pathway, the lectin pathway or the alternative pathway.

Thus, the method of the invention is able to identify the convertases complexes in tissue and biological specimen and be used to discriminate between a disorder associated with an aberration of the classical/lectin pathway and a disorder associated with an aberration of the alternative pathway.

The combined determination of the interaction of (i) C4b with C2b and (ii) C3b with Bb allows a particularly precise determination whether a disease associated with the complement system is due to aberrations of the classical/lectin pathway or the alternative pathway.

Typically, an increased amount of C4b/C2b complexes compared to a control is indicative for a disorder associated with the classical/lectin pathway. In some embodiments, the increased amount of C4b/C2b complexes compared to a predetermined threshold may be indicative for a disorder associated with the classical/lectin pathway.

Correspondingly, the increased amount of C3b/Bb complexes compared to a control is indicative for a disorder associated with the alternative pathway. In some embodiments, the increased amount of C3b/Bb complexes above a predetermined threshold may be indicative for a disorder associated with the alternative pathway.

In specific embodiments, not encompassed by the wording of the claims, a value of the amount of C4b/C2b complexes divided by the amount of C3b/Bb complexes larger than 1 may be indicative for a disorder associated with an aberration of the classical/lectin pathway. Correspondingly, in embodiments not according to the claimed invention, a value of the amount of C3b/Bb complexes divided by the amount of C4b/C2b complexes larger than 1 may, in embodiments not according to the claimed invention, be indicative for a disorder associated with an aberration of the alternative pathway.

Alternatively, also not encompassed by the wording of the claims, an increased value of the amount of C4b/C2b complexes divided by the amount of C3b/Bb complexes compared to a control may be indicative for a disorder associated with an aberration of the classical/lectin pathway. Correspondingly, in embodiments not according to the claimed invention, an increased value of the amount of C3b/Bb complexes divided by the amount of C4b/C2b complexes compared to a control may be indicative for a disorder associated with an aberration of the alternative pathway.

The combined determination of (i) the interaction of Ig with C1q and of (ii) the interaction of MASP1/3 with MBL and/or MASP2 with MBL allows a particularly precise determination whether a disease associated with the complement system is due to aberrations of the classical pathway or the lectin pathway.

The combined combination of (i) the interaction of C4b with C2b, of (ii) the interaction of C3b with Bb, of (iii) the interaction of Ig with C1q and of (iv) the interaction of MASP1/3 with MBL and/or MASP2 with MBL allows a particularly precise determination whether a disease associated with the complement system is due to aberrations of the classical pathway, the lectin pathway or the alternative pathway.

In step (b) the detection of the presence of the respective complement complex may be determined by *in situ* proximity ligation in the sample. Alternatively, in step (b) the detection of the presence of the respective complement complex is carried out by fluorescence resonance energy transfer (FRET) or a related method that allows to show proximity of two proteins or by Dexter energy transfer.

Accordingly, typically in step (b) the detection of the presence of the respective complement complex is carried out by microscopy including light microscopy, such as bright field microscopy or fluorescence microscopy.

In step (b) the detection of the presence of the C3/C5 convertase complex may be determined by *in situ* proximity ligation in the sample. Alternatively, in step (b) the detection of the presence of the C3/C5 convertase complex is carried out by fluorescence resonance energy transfer (FRET) or a related method that allows to show proximity of two proteins or by Dexter energy transfer.

Accordingly, typically in step (b) the detection of the presence of the C3/C5 convertase complex is carried out by light microscopy, such as bright field microscopy or fluorescence microscopy.

In some embodiments measurement may be carried out in the biopsy sample. Thereby an assessment step for identifying regions that are useful for measurement can be avoided.

Alternatively, measurement is carried out in intact glomeruli which may lead to highly accurate results in some cases, especially in glomerulonephritis.

Typically, the disorder associated with the complement system is selected from the group consisting of kidney disease, cancer, autoimmunity, infection, degeneration and neuropathy. Examples of kidney diseases are systemic lupus erythematodes (SLE), thrombotic microangiopathy caused by atypical hemolytic uremic syndrome (aHUS), Shiga-toxin producing *E. coli* hemolytic uremic syndrome (STEC-HUS), secondary hemolytic uremic syndrome (secondary HUS), C3 glomerulopathy, C3 glomerulonephritis, dense deposit disease, post-/parainfectious glomerulonephritis, IgA nephropathy, cryoglobulinemia, and membranoproliferative glomerulonephritis (MPGN).

In some embodiments, the disorder associated with an aberration of the classical/lectin pathway is selected from the group consisting of cryoglobulinemia, membranous nephropathy and SLE, preferably SLE.

In some embodiments, the disorder associated with an aberration of the alternative pathway is aHUS,

post-/parainfectious glomerulonephritis and C3 glomerulopathy.

The sample may be selected from the group consisting of body fluid or tissue sample.

Examples of body fluids are urine, liquor, blood, plasma, serum, effusion and synovial fluid.

Preferably, the tissue sample is a kidney tissue sample, more preferably a kidney biopsy.

Preferably, the body fluid is blood, plasma or serum, more preferably serum.

Another aspect of the invention refers to an *in vitro* method for determining whether a patient with a disorder associated with the complement system will be responsive to a modulator of the classical/lectin pathway or the alternative pathway, comprising the following steps:

(a) providing a sample of the patient;
(b) detecting the presence of C3/C5 convertase complex comprising the steps:

(i) determining the amount of C4b/C2b complexes in the sample and
(ii) determining the amount of C3b/Bb complexes in the sample;

wherein an increased amount of C4b/C2b complexes indicates that the patient will be responsive to an inhibitor of the classical/lectin pathway and/or wherein an increased amount of C3b/Bb complexes indicates that the patient will be responsive to an inhibitor of the alternative pathway;
(c) predicting whether the patient will be responsive to the inhibitor of the classical/lectin pathway or alternative pathway based on the result of step (b).

Preferably, the modulator is an inhibitor.

An increased amount of C4b/C2b complexes indicates that the patient will be responsive to an inhibitor of the classical/lectin pathway and/or an increased amount of C3b/Bb complexes indicates that the patient will be responsive to an inhibitor of the alternative pathway or alternatively any complement targeting inhibitor, like also the terminal complement pathway.

In some embodiments, the method may further comprise the additional detection of Ig/C1q complex in the sample; and/or detection of the amount of MASP1/3/MBL complex and/or MASP2/MBL complex in the sample in order to determine whether the patient with a disorder associated with the complement system will be responsive to a modulator of the classical pathway or the lectin pathway.

Another aspect of the invention refers to an *in vitro* method for determining the disorder course of a patient with a disorder associated with the complement system, comprising the following steps:

(a) Providing samples of a subject of at least two time points of the disease course;
(b) Detecting the presence of C3/C5 convertase complex in the samples comprising the steps:

(i) Determining the amount of C4b/C2b complexes in the samples and
(ii) Determining the amount of C3b/Bb complexes in the samples;

wherein a decreased amount of C4b/C2b complexes in the sample of the later time point compared to the sample of the earlier time point indicates an amelioration of the disorder of the classical/lectin pathway and wherein an increased amount of C4b/C2b complexes in the sample of the later time point compared to the sample of the earlier time point indicates worsening of the disorder of the classical/lectin pathway; and wherein a decreased amount of C3b/Bb complexes in the sample of the later time point compared to the sample of the earlier time point indicates an amelioration of the disorder of the alternative pathway and/or an increased amount of C3b/Bb complexes in the sample of the later time point compared to the sample of the earlier time point indicates worsening of the disorder of the alternative pathway;
(c) Determining the disorder course based on the result of step (b).

A decreased amount of C4b/C2b complexes in the sample of the later time point compared to the sample of the earlier time point indicates an amelioration of the disorder of the classical/lectin pathway and an increased amount of C4b/C2b complexes in the sample of the later time point compared to the sample of the earlier time point indicates worsening of the disorder of the classical/lectin pathway.

A decreased amount of C3b/Bb complexes in the sample of the later time point compared to the sample of the earlier time point indicates an amelioration of the disorder of the alternative pathway and/or an increased amount of C3b/Bb complexes in the sample of the later time point compared to the sample of the earlier time point indicates worsening of the disorder of the alternative pathway.

Another aspect of the invention relates to an *in vitro* method for determining whether a candidate compound is suitable for the treatment of a disease like a disorder of the complement system, wherein the prediction of a suitable candidate compound is based on step (c), comprising the following steps:

(a) Incubating a sample of a subject with a candidate compound;
(b) Detecting the presence of C3/C5 convertase complex in the sample of step (a) comprising the steps:

(i) Determining the amount of C4b/C2b complexes in the sample and
(ii) Determining the amount of C3b/Bb complexes in the sample;

wherein, a decreased amount of C4b/C2b complexes compared to a control indicates that the candidate compound is suitable for the treatment of the disorder of the complement system and wherein a decreased amount of C3b/Bb complexes compared to a control compound indicates that the candidate compound is suitable for the treatment of the disorder of the complement system;
(c) Predicting whether a candidate compound is suitable for the treatment of a disorder of the complement system based on the result of step (b).

A decreased amount of C4b/C2b complexes compared to a control indicates that the candidate compound is suitable for the treatment of the disorder of the complement system. Accordingly, a decreased amount of C3b/Bb complexes compared to a control compound indicates that the candidate compound is suitable for the treatment of the disorder of the complement system.
In some embodiments, the method may further comprise the additional detection of Ig/C1q complex in the sample; and/or detection of the amount of MASP1/3/MBL complex and/or MASP2/MBL complex in the sample.
Another aspect of the invention refers to a kit comprising:

a) probes for detecting the complexes of C4b and C2b comprising:

- probe containing a moiety specifically binding C4b and a first signal generating moiety,
- probe containing a moiety specifically binding C2b and a second signal generating moiety,

b) probes for detecting complexes of C3b and Bb comprising:

- probe containing a moiety specifically binding C3b and a first signal generating moiety,
- probe containing a moiety specifically binding Bb and a second signal generating moiety.

In one embodiment, the kit further comprises
c) probes for detecting the complexes of Ig and C1q comprising:

- probe containing a moiety specifically binding Ig and a first signal generating moiety,
- probe containing a moiety specifically binding C1q and a second signal generating moiety.

In a further embodiment, the kit further comprises
d) probes for detecting the complexes of MASP1/3 and/or MASP2 and MBL comprising:

- probe containing a moiety specifically binding MASP1/3 and/or MASP2 and a first signal generating moiety,
- probe containing a moiety specifically binding MBL and a second signal generating moiety.

In some embodiments, the analyte binding moiety (also termed "moiety specifically binding") and the signal generating moiety are part of the same molecule. In alternative embodiments, the analyte binding moiety and the signal generating moiety are different molecules.
A moiety specifically binding a complement factor, such as C4b, C2b, C3b or Bb, may for example be an antibody and binding fragment thereof, a nanobody, a non-antibody protein scaffold protein (Vazquez-Lombardi R. et al.), an aptamer, a nucleotide based molecule and a small molecule.
In specific embodiments, the first and the second signal generating moieties build a FRET or Dexter energy transfer pair or by a related method. In other embodiments, the first and the second signal generating moieties build an *in situ* proximity ligation pair.

## FIGURE LEGENDS

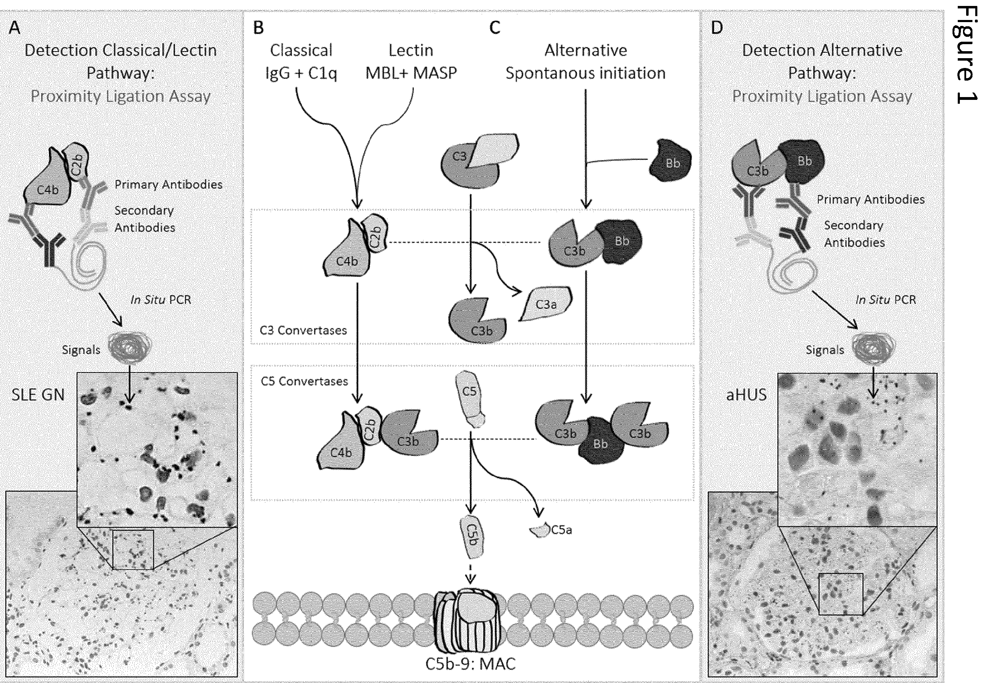

**Figure 1:** The complement cascade can be initiated by three pathways: the classical, lectin, and alternative pathway (B, C). Classical and lectin pathway activation results in assembly of C4b and C2b whereas the alternative pathway activation leads to assembly of C3b and Bb. Both C4bC2b and C3bBb are C3 convertases, which cleave surrounding C3, or after binding of another C3b molecule act as C5 convertases. The assembled complexes were detected by visualization of close proximity of their components by proximity ligation assay (A, D). Primary antibody binding was followed by the application of secondary antibodies, which are linked to oligonucleotides. The latter form rings enabling an *in situ* polymerase chain reaction with labeled probes resulting in a brown dot like signal for each C4b/C2 (A, bottom) and C3b/Bb (D, bottom) pair.

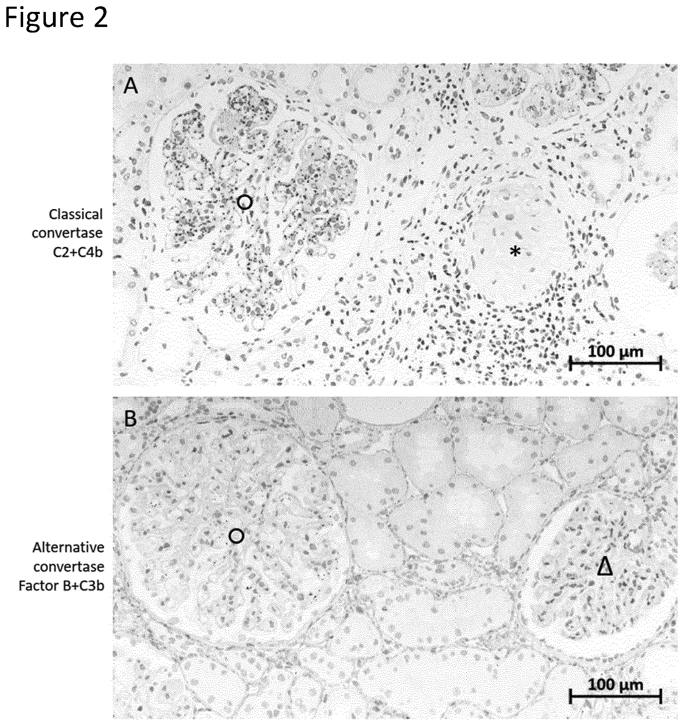

**Figure 2:** Exemplary depiction of glomeruli included and excluded from analysis.

A) Proximity ligation assay for classical/lectin pathway C3/C5-convertase in an SLE case. Note a preserved glomerulus with numerous signals included into the analysis (◦) and a representative scarred glomerulus without signals (*) excluded from analysis.
B) Proximity ligation assay for alternative pathway C3/C5-convertase in an aHUS case. Note a preserved glomerulus with signals included into the analysis (◦) and a representative collapsed glomerulus without signals (Δ) excluded from analysis.

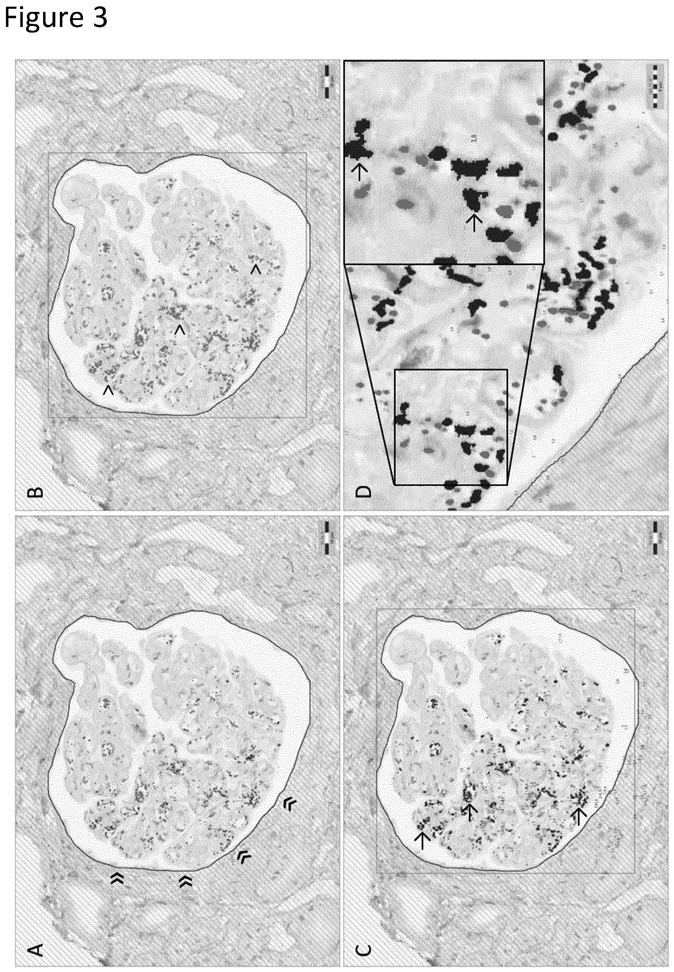

**Figure 3:** Overview of the computer-assisted quantification of signals in glomeruli.

A) Glomerulus of an aHUS case as region of interest (») for subsequent analysis. Note the abundant brown dot-like proximity ligation assay signals for the alternative pathway C3/C5-convertase inside the glomerular capillary lumens.
B) Detection of proximity ligation assay signals inside the defined region of interest. Note the green mark-up of the signals (>) according to the set parameters.
C), D) Automatized splitting of several confluent or overlapping signals into single signals (→). Note dark-green mark-up of the overlapping signals (→) and the number of estimated signals inside the respective area, subsequently rounded up, printed below the signals.

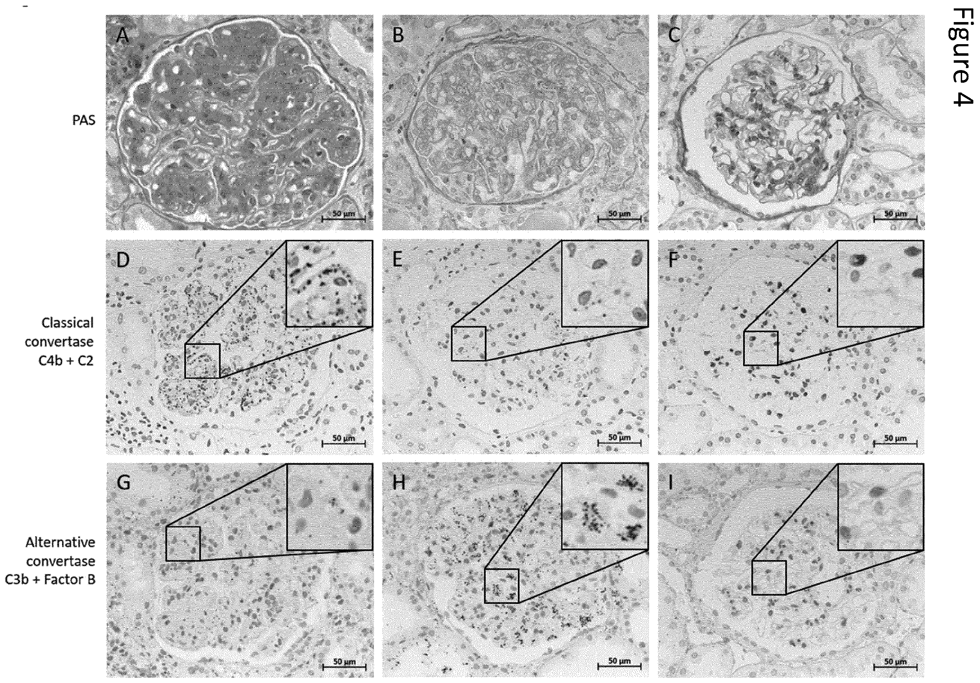

**Figure 4:** Proximity ligation assay for C4b/C2 and C3b/Factor B

A - C) PAS reaction of lupus nephritis (SLE GN), thrombotic microangiopathy due to atypical hemolytic syndrome (aHUS) and zero hour transplant biopsy as controls.
D- F) Proximity ligation assay for C4b/C2 of systemic lupus erythematosus (SLE), atypical hemolytic uremic syndrome (aHUS) and zero hour biopsies. Note brown dot-like PLA signals indicating assembly of the C3/C5 convertase of the classical/lectin pathway, most abundant in SLE (D) being located within the mesangium and along the peripheral glomerular capillary walls.
H - I) Proximity ligation assay for C3b/Factor B of systemic lupus erythematosus (SLE), atypical hemolytic uremic syndrome (aHUS) and zero hour biopsy. Note brown dot-like PLA signals indicating assembly of the C3/C5 convertase of the alternative pathway with most signals in aHUS (H) being located within the glomerular capillary lumens.

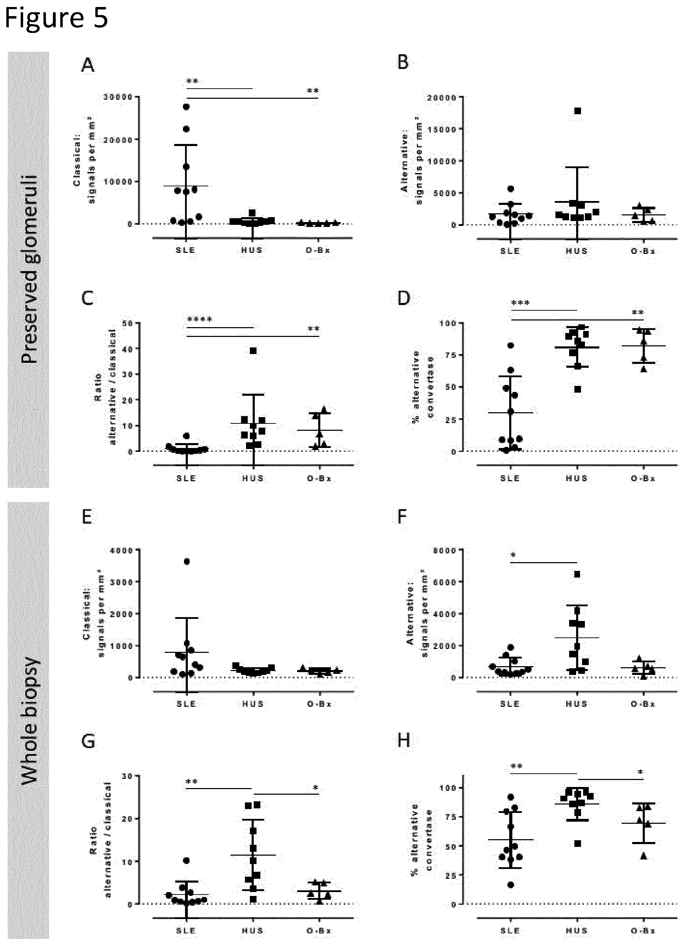

**Figure 5:** Results of the calculated signals in the preserved, presumably functional glomeruli only (A-D) and in whole biopsies (E-H). The classical convertases show significantly higher densities in SLE cases, as compared to HUS cases or zero hour transplant biopsies (O-Bx) taken as controls (A and E). In contrast, densities of the alternative convertase, as well as the alternative/classical pathway ratios were higher in HUS biopsies (B and F; C and G). The percentage of alternative pathway signals of all signals is lower in SLE cases compared to HUS cases and controls (D and H). Two-tailed Mann Whitney test was used to evaluate differences between the values (*=p<0.05; **=p<0.01; ***=p<0.001; ****=p<0.0001).

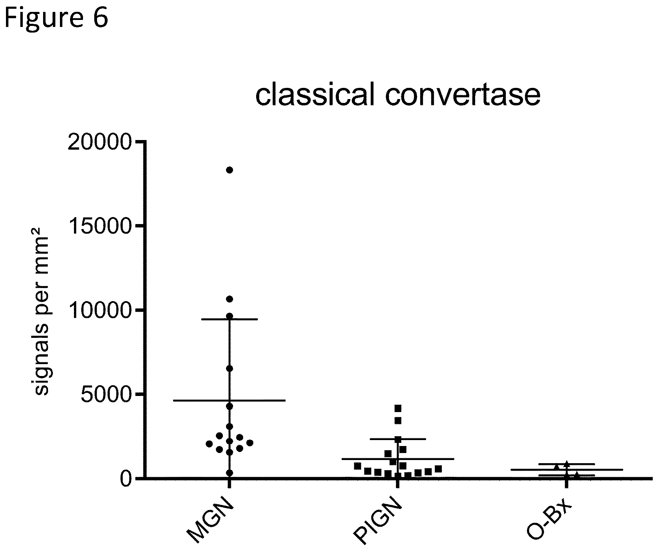

**Figure 6:** Results of proximity ligation assays for C4b/C2 in membranous nephropathy (MGN, n=15) and post-/parainfectious glomerulonephritis (PIGN, n=16). Nearly all cases of MGN show an enhanced signal density (average numbers of signals per mm2 glomerular area per case) for the assembly of the classical convertases compared to zero hour transplant biopsies (O-Bx) as normal controls. In contrast, only few cases of PIGN reveal an enhanced signal density. These preliminary results fit well into the hypotheses that in MGN subepithelial immune complexes initiate the classical complement pathway whereas in most cases of PIGN a direct activation of the alternative pathway is more likely.

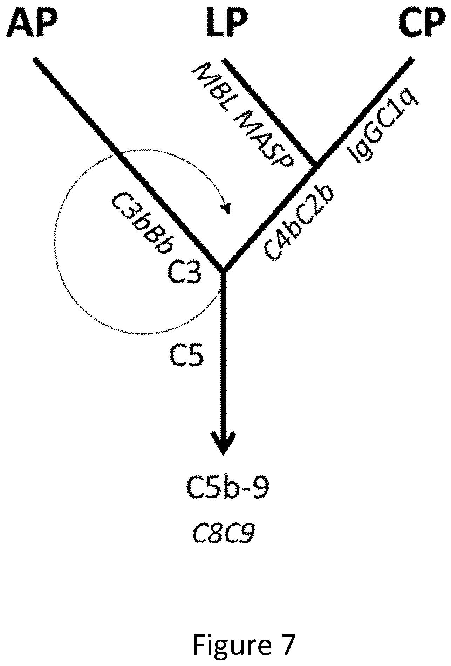

**Figure 7:** Simplified, schematic depiction of the complement system (AP = alternative pathway, LP = lectin pathway, CP = classical pathway). Molecules used for the proximity ligation assays mapped to the respective pathways are indicated.

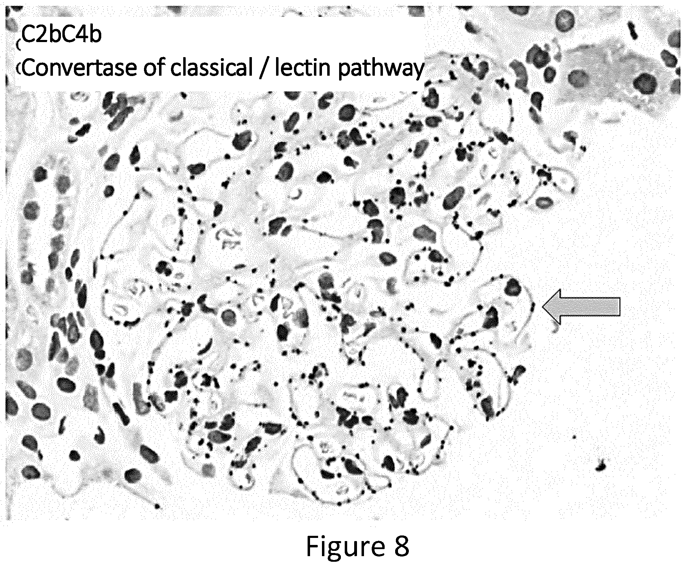

**Figure 8:** Example of the detection of C2/C4b signals for the classical/lectin pathway C3/C5 convertases. The brightfield proximity ligation method allows the evaluation of the signals within the microanatomical context. In the case of membranous nephropathy the signals are detected in the subepithelial space at the outer aspects of the glomerular basement membrane, as expected (arrow).

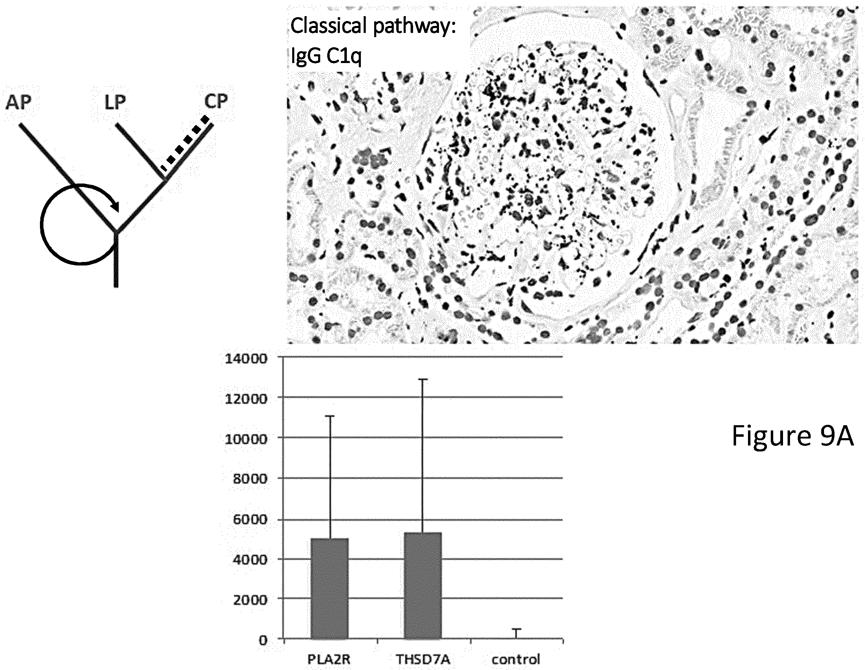

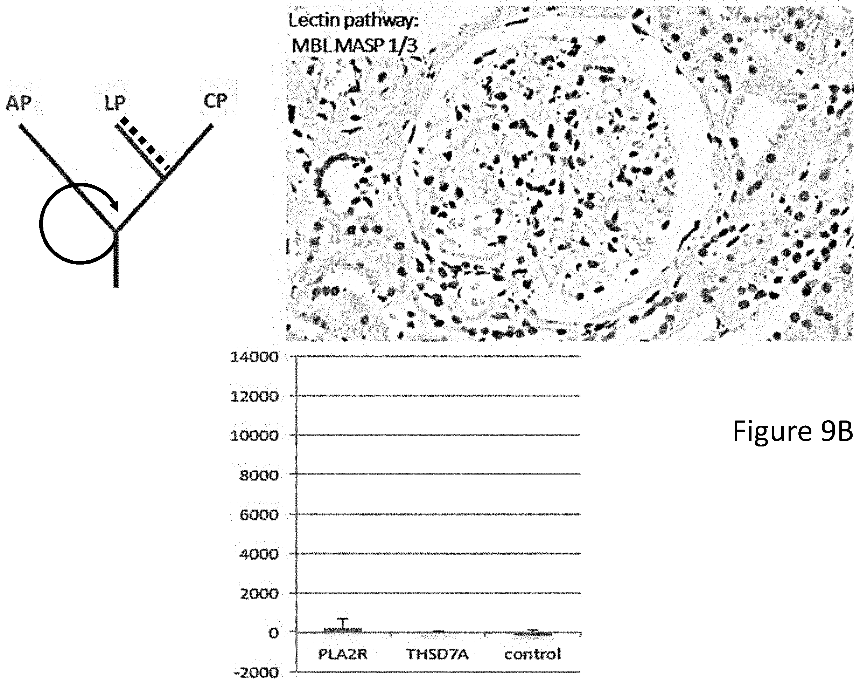

**Figure 9:** Scheme, representative light microscopic image, and results for A) IgG/C1q (classical pathway) and B) MBL/MASP1/3 (lectin pathway) in membranous nephropathy.

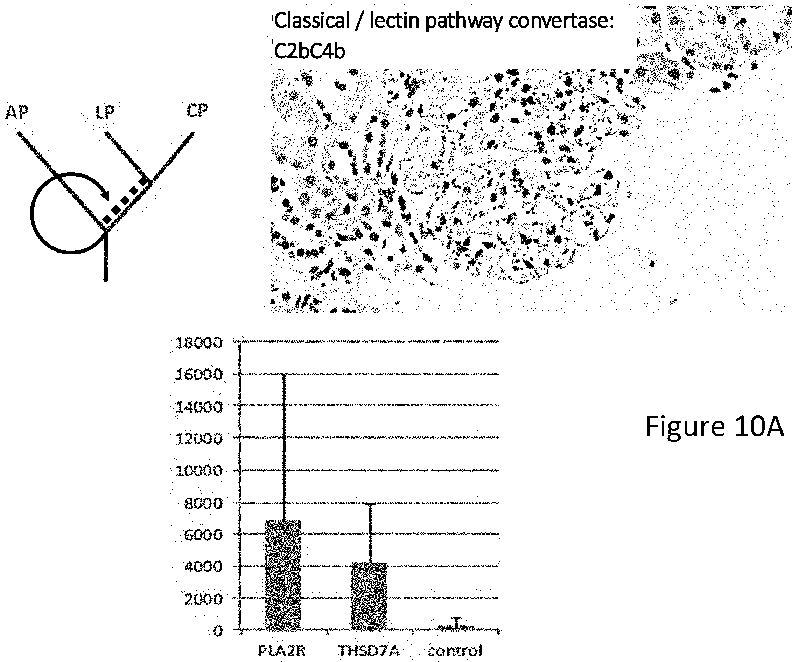

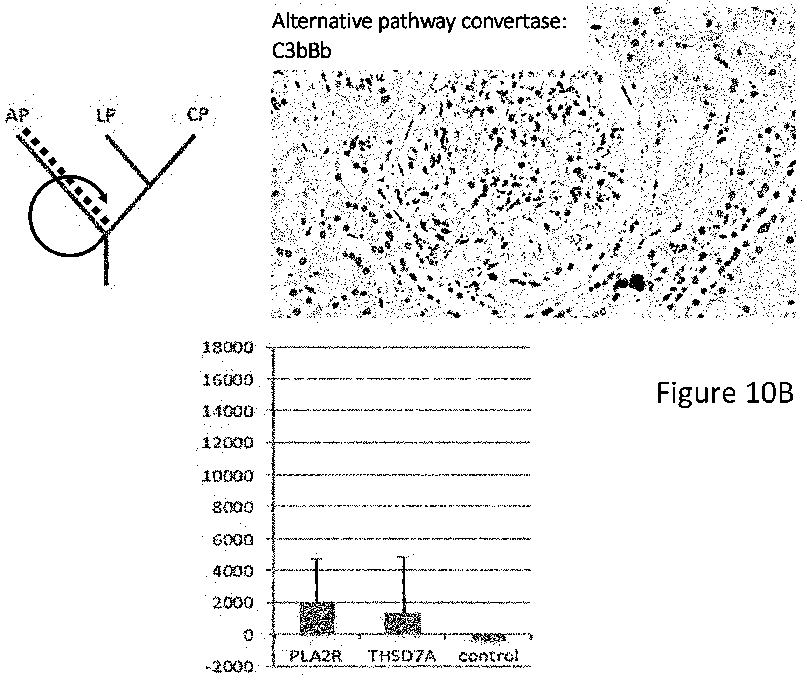

**Figure 10:** Scheme, representative light microscopic image, and results for A) C2/C4b (classical/lectin pathway) and B) C3b_B (alternative pathway) in membranous nephropathy.

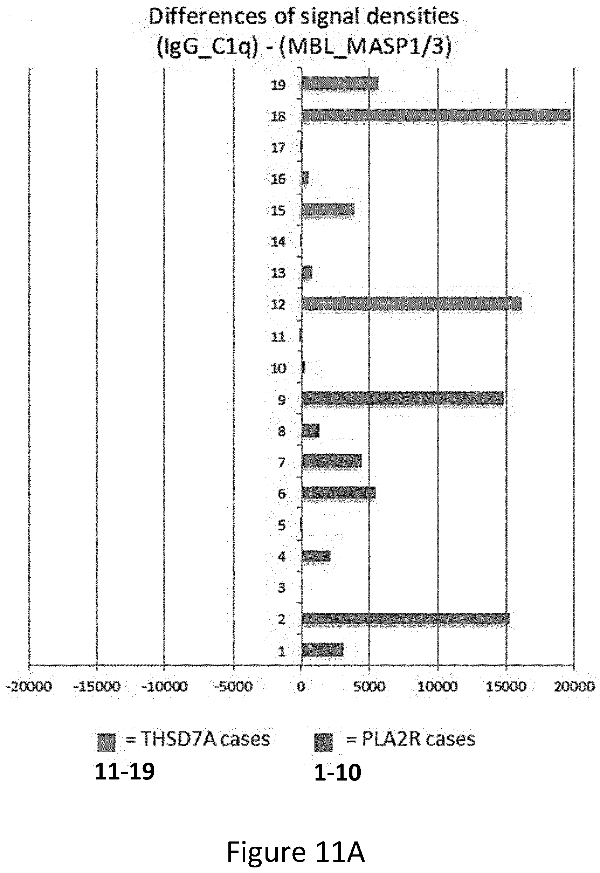

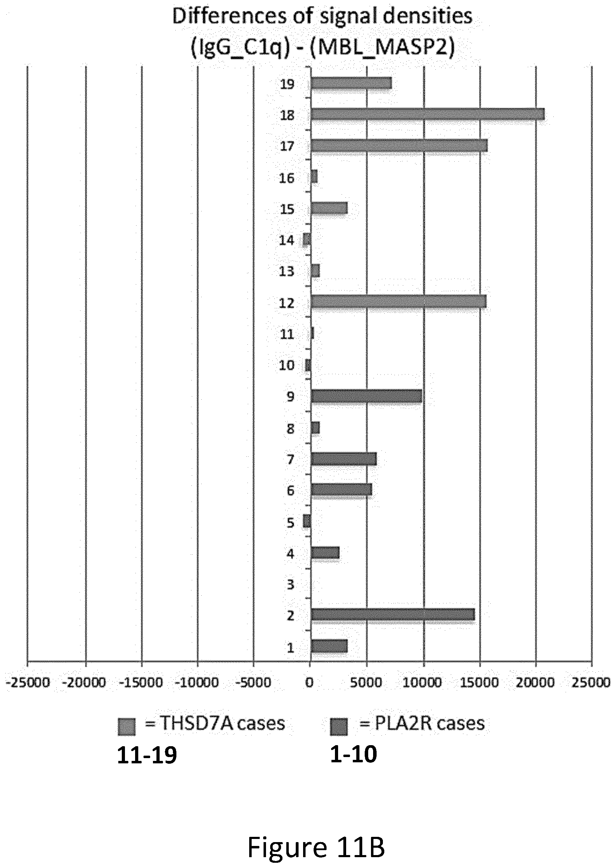

**Figure 11:** Differences of signal densities [signals per $mm^2$ glomerular area] after substraction of the respective tubulointerstitial background signal densities for the initiation phase. Substraction of A) MBL/MASP1/3 or B) MBL/MASP2 results from IgG/C1q results: one bar represents one case (blue=THSD7A, red=$PLA_2R$); a bar extending to the right with positive values depicts a case with a higher signal density for IgG/C1q (classical) than MBL/MASP1/3 or MBL/MASP2 (lectin).

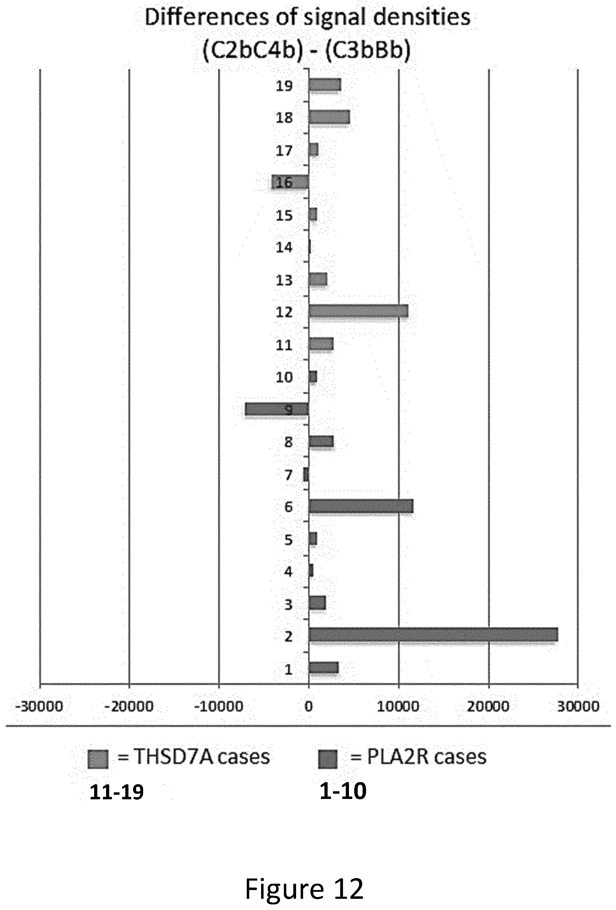

**Figure 12:** Differences of signal densities [signals per $mm^2$ glomerular area] after substraction of the respective tubulointerstitial background signal densities for the complement convertases. Substraction of C3b/B results from C2/C4b results: one bar represents one case (blue=THSD7A, red=$PLA_2R$); a bar extending to the right with positive values depicts a case with a higher signal density for C2/C4b (classical/lectin) than C3b/B (alternative).

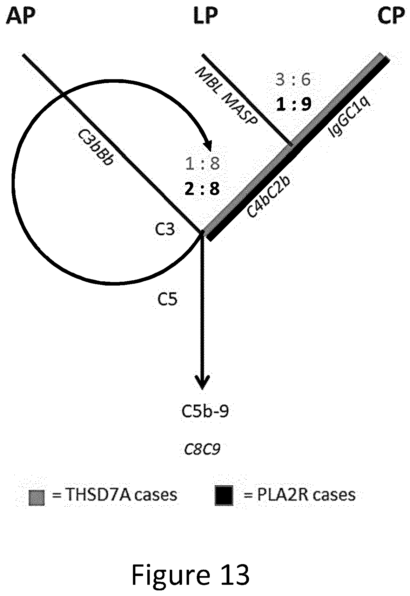

**Figure 13:** Schematic depiction of the complement mapping results for membranous nephropathy: Higher signal densities for IgG/C1q in 6 of 9 (THSD7A) and 9 of 10 ($PLA_2R$) cases as well as higher signal densities for C2/C4b in 8 of 9 (THSD7A) and 8 of 10 ($PLA_2R$) cases indicate predominant complement activation in membranous nephropathy via the classical pathway.

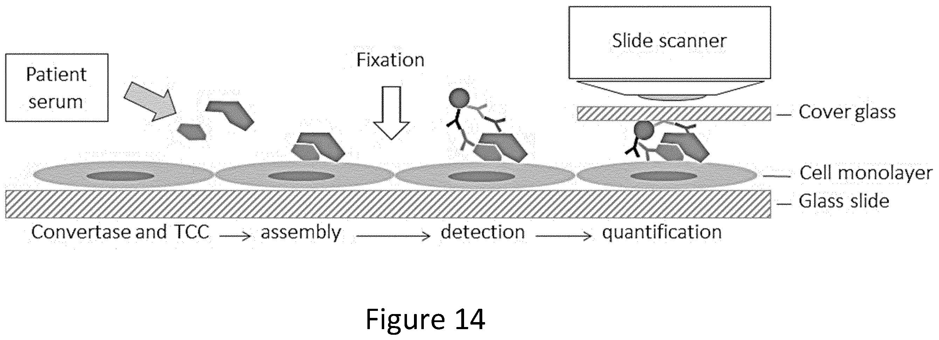

**Figure 14:** Schematic workflow of an assay to estimate the serum capacity to activate complement.

## DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of $\pm 10\%$, and preferably of $\pm 5\%$.

Technical terms are used by their common sense. If a specific meaning is conveyed to certain terms, definitions of terms will be given in the following in the context of which the terms are used.

C3 convertase is a protein complex comprising several protein subunits that acts as serine protease converting the protein C3 to C3a and C3b fragments. In particular embodiments, the term C3 convertase denotes two different protein complexes that can be distinguished by the present invention: (i) the C3 convertase of the classical/lectin pathway that contains the two proteins C4b and C2b; (ii) the C3 convertase of the alternative pathway that contains Bb and one C3b subunit.

C5 convertase is a protein complex comprising several proteins that acts as serine protease converting the protein C5 to C5a and C5b fragments. In particular embodiments, the term C5 convertase denotes two different protein complexes that can be distinguished by the present invention: (i) the C5 convertase of the classical/lectin pathway that contains the two proteins C4b and C2b and C3b; (ii) the C5 convertase of the alternative pathway that contains the protein Bb and two C3b proteins.

C3/C5 convertase complex denotes protein complexes that contain at least the components of the C3 convertase, i.e. (i) the C3 convertase of the classical/lectin pathway that contains the two proteins C4b and C2b; (ii) the C3 convertase of the alternative pathway that contains Bb and one C3b subunit, and may further contain (i) C3b resulting in the C5 convertase of the classical/lectin pathway or (ii) a second C3b subunit resulting in the C5 convertase of the alternative pathway.

C3 denotes human complement protein 3.

C4 denotes human complement protein 4.

C5 denotes human complement protein 5.

C2b also (formerly) termed C2a as used herein refers to the dissociated protein which is capable of binding to C4b.

C4b denotes the cleavage fragment of the complement component C4.

C3b denotes the cleavage fragment of the complement component C3.

Bb denotes the cleaved fragment/cleavage product of complement factor B.

C4b, C2b, C3b and Bb may for example be detected by commercially available antibodies such as anti-C3b antibody (mouse, Abcam, Cambridge, UK), anti-Factor B antibody (ANTI-CFB antibody produced in rabbit, HPA001832-100UL, Sigma Aldrich, Merck Darmstadt, Germany) anti-C4b antiserum (1:500, rabbit, Abcam, Cambridge, UK) and anti-C2 antibody (E-7) (1:10, mouse, Santa Cruz, sc-373809, Dallas, USA)

Ig/C1q complexes include immunoglobulin proteins (Ig) of all classes and subclasses, i.e. IgG with subfragments IgG1, IgG2, IgG3, IgG4, as well as IgA, IgA1, IgA2, IgE, IgD and IgM. The Igs associate with C1 the complement component of the classical pathway to initiate complement.

MASP1/3/MBL complex and MASP2/MBL complex: MASP1, MAPS2, MASP3 are pattern recognition proteins that interact with Mannan binding lectin (MBL) and initiate the lectin pathway of complement.

The terms "convertase" and "convertase complex" are used interchangeable herein.

A first aspect of the invention refers to an in vitro method for classifying a disorder associated with the complement system, comprising the following steps:

(a) providing a sample of a subject;

(b) detecting the presence of C3/C5 convertase complex comprising the steps:

  (i) determining the amount of C4b/C2b complexes in the sample and

  (ii) determining the amount of C3b/Bb complexes in the sample;

wherein an increased amount of C4b/C2b complexes compared to a control or predetermined threshold is indicative for a disorder associated with the classical/lectin pathway and wherein an increased amount of C3b/Bb complexes compared to a control or predetermined threshold is indicative for a disorder associated with the alternative pathway;

(c) classifying the disorder associated with the complement system based on the result of step (b).

In a preferred embodiment, step b) further comprises the following steps:

(i) determining the amount of Ig/C1q complex in the sample; and/or

(ii) determining the amount of MASP1/3/MBL complex and/or MASP2/MBL complex in the sample.

The subject may be a mammal, such as a rodent (e.g. a mouse or rat), cat, dog, leporid (e.g. rabbit) or a primate, such as a monkeys, human. Preferably, the subject is a mouse or a human. More preferably, the subject is a human. The subject may be a healthy or suffering from a disease associated with the complement system.

Determining the amount of C4b/C2b complexes in the sample and determining the amount of C3b/Bb complexes in the sample means detecting the complexes of C4b and C2b and detecting complexes of C3b with Bb.

An increased amount of C4b/C2B complexes or C3b/Bb complexes refers in particular to a higher number of C4b/C2b complexes or C3b/Bb complexes, in particular to a higher number of signal spots each representing in general one C4b/C2b complex or C3b/Bb complex; in some embodiments it may also refer increased signal spots or spots with increased signal intensity, that may represent a higher number of complexes compared to smaller signal spots or spots with less signal intensity which may represent a smaller number of complexes.

Vice versa, a decreased amount of C4b/C2B complexes or C3b/Bb complexes refers in particular to a lower number of C4b/C2b complexes or C3b/Bb complexes, in particular to a lower number of signal spots each representing in general one C4b/C2b complex or C3b/Bb complex; in some embodiments it may also refer to decreased signal spots or spots with decreased signal intensity, that may represent a lower number of complexes compared to larger signal spots or spots with higher signal intensity which may represent a higher number of complexes.

The skilled person understands that the with higher resolutions each spot may represent a single complex while in a lower resolution a spot, in particular an enlarged spot or a spot with higher intensity may represent several complexes. The skilled person can differentiate between these two scenarios; There may be settings in which both scenarios occur in one sampled.

"Determining the amount of complexes" means measuring a signal representative for the amount of the respective complement complex.

"Determining the amount of C4b/C2b complexes" or "determining the amount of C3b/Bb complexes" means measuring a signal representative for the amount of C4b/C2b complexes or C3b/Bb complexes.

Aberration in the context of the present invention refers to an altered activity. Thus "aberration of the classical/lectin pathway" means "altered activity of the classical/lectin pathway". Accordingly "aberration of the alternative pathway" means "altered activity of the alternative pathway". "Aberration of the classical pathway" means "altered activity of the classical pathway". "Aberration of the lectin pathway" means "altered activity of the lectin pathway".

Classifying the disorder of the complement system allows a more detailed specification of the disorder, in particular the classification, also termed discrimination, whether the disorder is associated with an aberration of the classical/lectin pathway and/or the diagnosis whether the disorder is associated with an aberration of the alternative pathway. **In** preferred embodiments, the classification discriminates between a disorder associated with an aberration of the classical/lectin pathway and a disorder associated with an aberration of the alternative pathway.

The skilled person understands that the methods described herein are *in vitro,* in particular *in situ,* methods. Therefore, diagnostic methods practiced on the human or animal body are not encompassed by the present description.

In some embodiments, the increased amount of C4b/C2b complexes compared to a control is indicative for a disorder associated with the classical/lectin pathway. Accordingly, the increased amount of C4b/C2b complexes compared to a predetermined threshold is indicative for a disorder associated with the classical/lectin pathway.

The skilled person is aware of methods for determining a threshold for the interaction of C4b and C2b that indicates whether a disorder is associated with the classical/lectin pathway. The threshold may be determined in a study with an appropriate number of patients.

For example, the threshold may be determined by the mean value of 30 healthy persons plus two standard deviations.

In some embodiments, the increased amount of C3b/Bb complexes compared to a control is indicative for a disorder associated with the alternative pathway. In other embodiments, the increased amount of C3b/Bb complexes above a predetermined threshold is indicative for a disorder associated with the alternative pathway.

In specific embodiments, not encompassed by the wording of the claims, a value of the amount of C3b/Bb complexes divided by the amount of C4b/C2b complexes larger than 1 is indicative for a disorder associated with an aberration of the alternative pathway.

Preferably, in this embodiment not encompassed by the wording of the claims, the value of the amount of C3b/Bb complexes divided by the amount of C4b/C2b complexes is a ratio larger than 2, more preferably larger than 3, even more preferably larger than 4, most preferably larger than 5, such as larger than 10.

In some embodiments, not encompassed by the wording of the claims, an increased value of the amount of C3b/Bb complexes divided by the amount of C4b/C2b complexes compared to a control is indicative for a disorder associated with an aberration of the alternative pathway.

The term "control" as used herein refers to a sample, such as a kidney biopsy of an individual which has no aberrations of the complement system, in particular not aberrations of the classical/lectin pathway and the alternative pathway. The skilled person understands that also an average of several such samples could is understood as control. The control may be for example a "zero-hour biopsy" of the same tissue as the sample to be tested. The term "zero-hour biopsy" as used herein refers to a biopsy that is taken from a (presumably healthy) transplanted kidney at the time of transplantation (time-point "zero-hour").

In some embodiments, not encompassed by the wording of the claims, a value of the amount of C4b/C2b complexes divided by the amount of C3b/Bb complexes larger than 1 is indicative for a disorder associated with an aberration of the classical/lectin pathway.

In step (b) the detection of the presence of the complement complex, e.g., the C3/C5 convertase complex, may be determined by any method in the art useful the detection of in situ protein interaction, for example by in situ proximity ligation, by fluorescence resonance energy transfer (FRET) or by Dexter energy transfer. Thus, in some embodiments, in step (b) the detection of the presence of the complement complex, e.g., the C3/C5 convertase complex, may be carried out by light microscopy, such as light microscopy is bright field microscopy or fluorescence microscopy.

FRET is based on the transfer of energy between two fluorophores, i.e. a donor and an acceptor, when these fluorophores are in close proximity. When the two fluorophores come close enough to each other, excitation of the donor by an energy source triggers an energy transfer towards the acceptor, which in turn emits specific fluorescence at a given wavelength. The skilled person is aware of FRET imaging techniques and how to adapt them (Jares-Erijman, Nature Biotechnology 21, 1387-1395 (2003)). In the present case, for the detection of the C3/C5 convertase of the classical/lectin pathway, a probe containing the analyte binding moiety and the signal generating moiety, such as an antibody targeting C4b linked to a FRET donor or FRET acceptor, may be used in combination with a further probe, such as an antibody targeting C3b and containing the respective counterpart of the FRET pair. Alternatively, probes, in which the analyte binding moiety and the signal generating moiety are separate molecules may be use. For example, the molecules containing the analyte binding moieties may be primary antibodies targeting C4b and C2b which may be used in combination with signal generating moieties, such as secondary antibodies containing an FRET donor, the other an FRET acceptor. Correspondingly, for the detection of the C3/C5 convertase of the alternative pathway, a probe, such as an antibody linked to an FRET donor or FRET acceptor would be used to detect C3b and a further probe, such as an antibody targeting Bb fused to the respective counterpart of the FRET pair. Alternatively, probes having separate molecules for detection and signal generation, such as primary antibodies targeting C3b and Bb in combination with secondary antibodies containing a FRET donor and a FRET acceptor, respectively, may be used.

The proximity ligation assay is commercially available, e.g. the Duolink® assay provided by SIGMA-Aldrich 8USA). The assay is described inter alia in Söderberg et al.. As described in the method section, for the detection of the C3/C5 convertase of the classical pathway, probe molecules containing the analyte binding moiety, such as primary antibodies targeting C4b and C2b may be used in combination with probe molecules containing the signal generating moiety, i.e. proximity ligation assay secondary antibodies. Correspondingly, for the detection of the C3/C5 convertase of the alternative pathway secondary proximity ligation antibodies targeting the primary antibodies targeting C3b and Bb may be used. In principle, alternatively, also probes containing both the analyte binding moiety that target the complex subunits (i.e. C4b, C2b, C3b and Bb) and the signal generating moiety containing the proximity ligation assay domains can be used.

In some embodiments, the measurement may be carried out in the whole biopsy sample or in intact glomeruli.

The term whole biopsy sample refers to the entire sample without any discrimination of substructures such as glomeruli.

The term "measurement is carried out in intact glomeruli" means that only glomeruli that are intact are measured. The term intact glomeruli" refers to glomeruli of normal appearance, i.e. not scarred, not sclerosed, not necrotic, and not collapsed.

The term "disorder associated with the complement system" refers to diseases due to aberrations of the complement system. In particular, to diseases that lead to an aberrant number of C3/C5 convertases of the classical pathway or to an aberrant number of C3/C5 convertases of the alternative pathway.

In some embodiments, the disorder associated with the complement system is selected from the group consisting of kidney disease, cancer, autoimmunity, infection, degeneration and neuropathy. The kidney disease may be selected from the group consisting of systemic lupus erythematodes (SLE), atypical hemolytic uremic syndrome (aHUS), Shiga-toxin producing E. coli hemolytic uremic syndrome (STEC-HUS), secondary hemolytic uremic syndrome (secondary HUS), thrombotic microangiopathy caused by atypical hemolytic uremic syndrome (aHUS), C3 glomerulopathy, cryoglobulinemia, glomerulonephritis and dense deposit disease.

The glomerulonephritis may be selected from the group IgA nephritis, ANCA-associated nephritis, C3 glomerulonephritis, dense deposit disease, membranoproliferative glomerulonephritis (MPGN), membranous nephropathy, post-/parainfectious glomerulonephritis.

In some embodiments, the disorder associated with an aberration of the classical/lectin pathway is selected from the group consisting of cryoglobulinemia, membranous nephropathy and SLE, preferably SLE.

In other embodiments, the disorder associated with an aberration of the alternative pathway is aHUS post-/parainfectious glomerulonephritis and C3 glomerulopathy.

Typically, the sample is selected from the group consisting of body fluid or tissue sample. In some embodiments, the body fluid is selected from the group consisting of urine, liquor, blood, plasma, serum, effusion and synovial fluid. Preferably, the tissue sample is a kidney tissue sample.

Another aspect of the invention refers to an *in vitro* method for determining whether a patient with a disorder associated with the complement system will be responsive to a modulator of the classical/lectin pathway or alternative pathway, comprising the following steps:

(a) providing a sample of the patient;
(b) detecting the presence of C3/C5 convertase complex comprising the steps:

(i) determining the amount of C4b/C2b complexes in the sample and
(ii) determining the amount of C3b/Bb complexes in the sample;

wherein an increased amount of C4b/C2b complexes indicates that the patient will be responsive to an inhibitor of the classical/lectin pathway and/or wherein an increased amount of C3b/Bb complexes indicates that the patient will be responsive to an inhibitor of the alternative pathway;
(c) predicting whether the patient will be responsive to the modulator of the classical/lectin pathway or alternative pathway based on the result of step (b).

In preferred embodiments, the modulator is an inhibitor.
The term "modulator", in particular "inhibitor", includes small molecules, antibodies and binding fragments thereof, non-antibody protein scaffold proteins, aptamers and nucleotide based molecules.
In some embodiments, the inhibitor is selected from the group consisting of Eculizumab and Ravulizumab (C5 inhibitors, both Alexion); C1 inhibitors Berinert and Cinryze; soluble C5a peptide binding mAb (IFX-1; InflaRx); C5a receptor antagonist (Avacopan, Chemocentryx); OMS 721 also termed, narsoplimap, a MASP2 inhibitor (Omeros); Amy 101 (Amyndas); APL2 a C3 targeting peptide (Apellis); Danicopan (ACH-4471), a Factor D binding antagonist (Achillion); LNP023 a Factor B blocking compound (Novartis).
An increased amount of C4b/C2b complexes indicates that the patient will be responsive to an inhibitor of the classical/lectin pathway and/or an increased amount of C3b/Bb complexes indicates that the patient will be responsive to an inhibitor of the alternative pathway.
Another aspect of the invention refers to an *in vitro* method for determining the disorder course of a patient with a disorder associated with the complement system, comprising the following steps:

(a) Providing samples of a subject of at least two time points of the disease course;
(b) Detecting the presence of C3/C5 convertase complex in the samples comprising the steps:

(i) Determining the amount of C4b/C2b complexes in the samples and/
(ii) Determining the amount of C3b/Bb complexes in the samples;

wherein a decreased amount of C4b/C2b complexes in the sample of the later time point compared to the sample of the earlier time point indicates an amelioration of the disorder of the classical/lectin pathway and wherein an increased amount of C4b/C2b complexes in the sample of the later time point compared to the sample of the earlier time point indicates worsening of the disorder of the classical/lectin pathway;
and wherein a decreased amount of C3b/Bb complexes in the sample of the later time point compared to the sample of the earlier time point indicates an amelioration of the disorder of the alternative pathway and/or an increased amount of C3b/Bb complexes in the sample of the later time point compared to the sample of the earlier time point indicates worsening of the disorder of the alternative pathway;

(c) Determining the disorder course based on the result of step (b).

A decreased amount of C4b/C2b complexes in the sample of the later time point compared to the sample of the earlier time point indicates an amelioration of the disorder of the classical/lectin pathway and an increased amount of C4b/C2b complexes in the sample of the later time point compared to the sample of the earlier time point indicates worsening of the disorder of the classical/lectin pathway.
A decreased amount of C3b/Bb complexes in the sample of the later time point compared to the sample of the earlier time point indicates an amelioration of the disorder of the alternative pathway and/or an increased amount of C3b/Bb complexes in the sample of the later time point compared to the sample of the earlier time point indicates worsening of the disorder of the alternative pathway.
The method may further comprise the additional detection of Ig/C1q complex in the sample; and/or detection of the amount of MASP1/3/MBL complex and/or MASP2/MBL complex in the sample.
Another aspect of the invention relates to an *in vitro* method for determining whether a candidate compound is suitable for the treatment of a disorder of the complement system, wherein the prediction of a suitable candidate compound is based on step (c), comprising the following steps:

(a) Incubating a sample of a subject with a candidate compound;

(b) Detecting the presence of C3/C5 convertase complex comprising the steps:

(i) Determining the amount of C4b/C2b complexes in the sample and
(ii) Determining the amount of C3b/Bb complexes in the sample;

wherein, a decreased amount of C4b/C2b complexes compared to a control indicates that the candidate compound is suitable for the treatment of the disorder of the complement system and wherein a decreased amount of C3b/Bb complexes compared to a control compound indicates that the candidate compound is suitable for the treatment of the disorder of the complement system;
(c) Predicting whether a candidate compound is suitable for the treatment of a disorder of the complement system based on the result of step (b).

A decreased amount of C4b/C2b complexes compared to a control indicates that the candidate compound is suitable for the treatment of the disorder of the complement system. Accordingly, a decreased amount of C3b/Bb complexes compared to a control compound indicates that the candidate compound is suitable for the treatment of the disorder of the complement system.
The methods described herein may be carried out with a sample that has been obtained from a subject or a patient.
Another aspect of the invention refers to a kit comprising:

a) probes for detecting the complexes of C4b and C2b comprising:

- probe containing a moiety specifically binding C4b and a first signal generating moiety,
- probe containing a moiety specifically binding C2b and a second signal generating moiety,

b) probes for detecting complexes of C3b and Bb comprising:

- probe containing a moiety specifically binding C3b and a first signal generating moiety,
- probe containing a moiety specifically binding Bb and a second signal generating moiety.

In one embodiment, the kit further comprises
c) probes for detecting the complexes of Ig and C1q comprising:

- probe containing a moiety specifically binding Ig and a first signal generating moiety,
- probe containing a moiety specifically binding C1q and a second signal generating moiety.

In a further embodiment, the kit further comprises
d) probes for detecting the complexes of MASP1/3 and/or MASP2 and MBL comprising:

- probe containing a moiety specifically binding MASP1/3 or MASP2 and a first signal generating moiety,
- probe containing a moiety specifically binding MBL and a second signal generating moiety.

In some embodiments, the analyte binding moiety and the signal generating moiety are part of the same molecule.
In alternative embodiments, the analyte binding moiety and the signal generating moiety are different molecules.
In specific embodiments, the first and the second signal generating moieties build a FRET /Dexter energy transfer pair. In other embodiments, the first and the second signal generating moieties build an *in situ* proximity ligation pair.

## EXAMPLES

Example 1

Ten kidney biopsies with lupus nephritis (SLE, class IV), nine cases with thrombotic microangiopathy (TMA) due to atypical hemolytic uremic syndrome, and five zero hour transplant biopsies (control) were analyzed. Preserved glomeruli were defined as regions of interest while scarred or collapsed glomeruli were excluded (Fig. S1 in the Supplementary Appendix). Proximity ligation signal densities (number of signals per glomerular area [$mm^2$]) were determined using image analysis software (hs analysis), and compared between the groups (Fig. S2 in the Supplementary Appendix).

As expected, SLE cases revealed higher densities of classical/lectin convertases within the glomerular mesangium and around capillary walls (median 7,685 signals/mm$^2$), as compared to TMA biopsies (median 393 signals/mm$^2$) and to control biopsies (median 207 signals/mm$^2$). In contrast, TMA cases showed a predominance of alternative convertases with most signals being located within the glomerular capillary lumens (median 3,032 signals/mm$^2$), as compared to SLE cases and control biopsies (median 1,329 signals/mm$^2$ and 1,418 signals/mm$^2$, respectively) (Fig. S3 and S4 in the Supplementary Appendix). Thus, we introduce the first methodological workflow for the visualization, differentiation and quantification of classical/lectin and alternative C3/C5 convertases directly within the tissue specimen. This new approach represents a tool to discriminate complement pathways in tissue and to show the dynamics of activation, enhancing diagnosis and potentially allowing future efficacy monitoring of individualized therapy.

Patients and Biopsies

From the archive of the pathology institute of the University Hospital Hamburg-Eppendorf (UKE), 10 cases of systemic lupus erythematodes nephritis class IV and 9 cases of atypical hemolytic uremic syndrome were selected. Five transplant zero hour biopsies were chosen as l control. The study was approved by the local ethics committee for Hamburg (Ethics Commission Hamburg, PV 5541). All work was carried out in accordance with the Declaration of Helsinki.

Methods

Immunohistochemistry and Proximity Ligation Assay

Slides (1$\mu$m) from paraffin embedded kidney biopsies were deparaffinized, pretreated and blocked using normal horse serum (Vector Industries S-2000). As pretreatment for C3b and Factor B detection Dako Target Retrieval Solution (S1699, Sigma-Aldrich, Darmstadt, Germany) was applied for 15 min at 120°C. Primary antibodies against C3b (1:1000, mouse) (Abcam, Cambridge, UK) and Factor B (1: 100, rabbit) (Sigma Aldrich, Darmstadt, Germany) were applied at 4° C for 16 hours. For detection of the classical C3/C5 convertase (C4b and C2) the tissue sections were pretreated with protease for 30 min at 40°C (P8038, Sigma-Aldrich, Darmstadt, Germany). Afterwards C4b antiserum (1:500, rabbit, Abcam, Cambridge, UK) and C2 antiserum (1:10, mouse, Santa Cruz, Dallas, USA) were applied at 4° C for 16 hours. Next, proximity ligation assay secondary antibodies against mouse (Duolink® In Situ PLA Probe Anti-Mouse Minus, Sigma Aldrich, Darmstadt, Germany) and rabbit (Duolink® In Situ PLA Probe Anti-Rabbit Plus, Sigma Aldrich, Darmstadt, Germany) antibodies were added. According to protocol the brightfield detection system (Duolink® In Situ Detection Reagents Brightfield, Sigma Aldrich, Darmstadt, Germany) was applied to the slides. The same primary antibodies were used on subsequent slides to visualize the deposition of C4b, C2, C3b and Factor B individually. For staining of C3 (1:3000, rabbit, polyclonal), C1q (1:1500, rabbit, polyclonal) (both DAKO, Eching, Germany) and IgG (1:7500, mouse, monoclonal) (Dianova, Hamburg, Germany), slides were pretreated with protease (15 min, 40°C) (P8038, Sigma-Aldrich, Darmstadt, Germany) and a standard APAAP-Protocol (monoclonal or polyclonal) was used. Detection was performed with the ZytoChem Plus AP Polymer System (POLAP-100, Zytomed, Berlin, Germany) using new fuchsin red chromogen and haematoxylin nuclear counterstain.

Quantification of proximity ligation assay signals

Densities of signals in glomeruli and in whole biopsies were analyzed using image analysis software (HSA KIT, HS Analysis GmbH, Karlsruhe, Germany). Scarred or collapsed glomeruli were excluded from glomerular analyses. With the software HSA KIT the PLA signals were detected, counted and measured using the Maximally Stable Extremal Regions (MSER) algorithm, which is described below. The following parameters were defined for this purpose: color ranges separating the foreground from the background of the signals within the biopsy as well as intervals for the intensity and for the size of the signals. Two categories of false positive signals occurred; intramembranous pseudo signals and intranuclear pseudo signals in cases with strong nuclear counterstaining. False positive intramembranous pseudo signals occurred only very rarely, so that they were negligible for the evaluation. In order to minimize the number of false positive intranuclear signals, a further parameter set was defined to determine the intensity and color values for negative signals. These negative signals are also detected via the MSER algorithm using an additional parameter set consisting of intensity and color values.

Negative signals and especially regions where positive and negative signals overlapped were excluded from the evaluation. A live overlay showing both the positive and negative signals allowed for easy parameter tuning until satisfactory values were found.

The MSER algorithm detects a number of features in an image. These MSERs are stable connected components that are chosen from a binary threshold representation of the image. The algorithm works as follows. A set of n equidistant threshold levels for the image signals $T = \{T_1..T_n\}$ is chosen. For every threshold level a pixel set $S_i = \{x : I(x) \leq T_i\}$ is generated from image $I(x)$ where $x = \begin{pmatrix} x_1 \\ x_2 \end{pmatrix}$ is a two-dimensional index. For every set $S_i$ connected regions $R_i \subset S_i$ are detected and arranged as a tree, $R_i$ is a child region of $R_i$+1 if $R_i \subset R_{i+1}$. The goal is now to select regions of maximal stability, in the sense that they do not change over different threshold levels. For that the algorithm assumes that regions that have a similar size as their child regions also do not change much in shape. Every region is assigned an instability score

$$z(R_i) = \frac{|R_i - R_i + 1|}{|R_i|}$$

The regions with minimal instability score are selected. Optionally the score can be modified to exclude regions that are too small ($|R_i|$ < *min*) or too big (c > *max*) in size:

$$z(R_i) = \begin{cases} \infty & \text{if } |R_i| < \min \text{or} |R_i| > \max \\ \dfrac{|R_i - R_{i+1}|}{|R_i|} & \text{otherwise} \end{cases}$$

Statistical analyses

Data were presented as mean ± standard deviation and median. A two-tailed Mann Whitney test was used to evaluate differences between the values. All statistical analyses were performed using R Software version 3.4.3 packages rcorr and ggplot2 (R Foundation for Statistical Computing, Vienna, Austria). Visualization of the statistical results was performed using the GraphPad Prism software (GraphPad Software, La Jolla California USA). $P < 0.05$ was considered statistically significant.

Example 2

The aim is to extend the method of *in-situ* visualization of C3/C5 convertases in order to dissect initiation of the lectin pathway and the classical pathway upstream as well as visualization and quantification of the membrane attack complex (C5b-9) downstream (see Fig. 7). At the end the combination provides a mapping of complement action in individual entities and patients.

Methods: Proximity ligation assays were performed for both convertases as described above. In addition, the same technique was applied for IgG with C1q for classical pathway initiation and for MASP1/3 with MBL as well as MASP2 with MBL for lectin pathway initiation . Brightfield proximity ligation assays were used and quantification of the signals was done as described. The method can also be applied to the detection of assembled C5b-9, e.g. by the detection of close proximity of C8 and C9. For the other reactions, the following antibodies were used:

| C3b/C3 [755] | abcam ab11871 | mouse mono | 1+1.000 |
|---|---|---|---|
| C2 (E-7) | Santa Cruz sc-373809 | mouse mono | 1+10 |
| MASP1/3 | bioss bs-1723R | rabbit poly | 1+100 |
| C1q | Dako A 013602-2 Biomol | rabbit poly | 1+1500 |
| CFB(r) | proteintech 10170-1-AP | rabbit poly | 1+20 |
| MASP2 | bioss bs-1980R | rabbit poly | 1+20 |
| MBL2 (3E7) | ThermoFisher MA1-40145 | mouse mono | 1+20 |
| C4b | abcam ab181241 | rabbit mono | 1+500 |
| IgG | Dianova 209-005-088 Jackson Immuno Research | mouse mono | 1+7500 |

Complement mapping was performed as a proof of concept study in 19 biopsy cases with membranous nephropathy (10 $PLA_2R$ associated and 9 THSD7A associated forms) and 9 zero hour transplant biopsies as normal controls.

Results: Specific signals were detected in membranous nephropathy cases at the expected location of the subepithial spaces at the outer aspects of the glomerular basement membranes (Fig. 8). Many signals for IgG/C1q could be detected but only very few signals for MBL/MASP1/3, pointing to the initiation of the complement system via the classical pathway (s. Fig. 9). Regarding the convertases very high signal densities for the classical/lectin convertase (C2bC4b) and fewer signals for the alternative pathway convertase could be seen (s. Fig. 10). Calculating the differences between the signal densities after substraction of the tubulointerstitial background signal densities revealed higher signal densities for IgG/Cq1 than MBL/MASP1/3 and MBL/MASP2 (Fig. 11) in all cases and higher signal densities for the classical/lectin convertase than for the alternative convertase in most cases (16 of 19; s. Fig. 12).

Conclusion: In membranous nephropathy complement is activated likely via the classical pathway (IgG/C1q and C2/C4b) in both THSD7A- and $PLA_2R$-associated cases (s. Fig. 13) with subsequent milder activation also of the alternative pathway convertase (C3bBb) probably via the amplification loop.

Example 3

In order to estimate the capacity of the serum of individual patients at a given time to activate complement, the assay as described above can also be applied to serum. For this purpose cell lines (i.e. HUVEC cells) could be grown as a monolayer in cell culture on a transparent microscope glass slide (see Fig. 14). Then different dilutions of patient serum are added into the supernatant. Complement activation will lead to assembly of the alternative complement C3 and C5 convertase and to the assembly of the terminal complement complex (TCC) C5b-9. The cells as well as the assembled convertases and TCCs are fixed after a certain time, e.g. using formaldehyde, in order to stop complement action before cell lysis. Subsequently, proximity ligation assays will be performed as described, the cells will be covered with a cover glass and quantification will be done as published (Person et al. 2020).

This technique provides a tool to monitor complement activation capacity of patient serum at the time of diagnosis and during therapy. Furthermore, it allows to test the efficacy of different complement targeting drugs in a given patient.

## REFERENCES

Thurman et al. Complement in kidney disease: core curriculum 2015. Am J Kidney Dis 2015;65:156-68.

Zipfel et al. The role of complement in C3 glomerulopathy. Mol Immunol 2015;67:21-30. Reddy et al. Breaking down the complement system: a review and update on novel therapies. Curr Opin Nephrol Hypertens 2017;26:123-8.

Ricklin D et al. The renaissance of complement therapeutics. Nat Rev Nephrol 2018;14:26-47.

Söderberg et al. Direct observation of individual endogenous protein complexes in situ by proximity ligation. (Nat Methods, 3, 995-1000(2006))

Vazquez-Lombardi et al. Challenges and opportunities for non-antibody scaffold drugs. Drug Discovery Today 2015;20(10):1271-1283.

## Claims

1. *In vitro* method for classifying a disorder associated with the complement system, comprising the following steps:

   (a) providing a sample of a subject;
   (b) detecting the presence of C3/C5 convertase complex in the sample of the subject comprising the following steps:

      (i) determining the amount of C4b/C2b complexes in the sample and
      (ii) determining the amount of C3b/Bb complexes in the sample,

   wherein an increased amount of C4b/C2b complexes compared to a control or predetermined threshold is indicative for a disorder associated with the classical/lectin pathway and wherein an increased amount of C3b/Bb complexes compared to a control or predetermined threshold is indicative for a disorder associated with the alternative pathway;
   (c) classifying the disorder associated with the complement system based on the result of step (b).

2. *In vitro* method according to claim 1, wherein the increased amount of C4b/C2b complexes compared to a predetermined threshold is indicative for a disorder associated with the classical/lectin pathway.

3. *In vitro* method according to any one of the preceding claims, wherein in step (b) the detection of the amount of the C3/C5 convertase complex is determined by *in situ* proximity ligation in the sample.

4. *In vitro* method according to any one of the preceding claims, wherein step b) further comprises the following steps:

(i) determining the amount of Ig/C1q complex in the sample; and/or
(ii) determining the amount of MASP1/3/MBL complex and/or MASP2/MBL complex in the sample.

5. *In vitro* method according to any one of the preceding claims, wherein the sample is selected from the group consisting of body fluid or tissue sample, wherein the sample is preferably a kidney tissue sample.

6. *In vitro* method according to any one of the preceding claims, wherein the sample is a plasma sample.

7. *In vitro* method according to any one of the preceding claims, wherein the disorder associated with the complement system is selected from the group consisting of kidney disease, cancer, autoimmunity, infection, degeneration and neuropathy.

8. *In vitro* method according to claim 7, wherein the kidney disease is selected from the group consisting of systemic lupus erythematosus (SLE), thrombotic microangiopathy caused by atypical hemolytic uremic syndrome (aHUS), Shiga-toxin producing *E. coli* hemolytic uremic syndrome (STEC-HUS), secondary hemolytic uremic syndrome (secondary HUS), C3 glomerulopathy, cryoglobulinemia, C3 glomerulonephritis, dense deposit disease, post-/parainfectious glomerulonephritis, IgA nephropathy, cryoglobulinemia, and membranoproliferative glomerulonephritis (MPGN).

9. *In vitro* method according to any one of the preceding claims, wherein the disorder associated with an aberration of the classical/lectin pathway is selected from the group consisting of cryoglobulinemia, membranous nephropathy and SLE, preferably SLE and wherein the disorder associated with an aberration of the alternative pathway is a HUS, post-/parainfectious glomerulonephritis and C3 glomerulopathy.

10. *In vitro* method according to any one of the preceding claims, wherein the sample is selected from the group consisting of body fluid or tissue sample.

11. *In vitro* method according to any one of the preceding claims, wherein the tissue sample is a kidney tissue sample.

12. *In vitro* method for determining whether a patient with a disorder associated with the complement system will be responsive to a modulator of the classical/lectin pathway or alternative pathway, comprising the following steps:

(a) Providing a sample of the patient;
(b) Detecting the presence of C3/C5 convertase complex in the sample of the patient comprising the steps:

(i) Determining the amount of C4b/C2b complexes in the sample and
(ii) Determining the amount of C3b/Bb complexes in the sample,

wherein an increased amount of C4b/C2b complexes indicates that the patient will be responsive to an inhibitor of the classical/lectin pathway and/or wherein an increased amount of C3b/Bb complexes indicates that the patient will be responsive to an inhibitor of the alternative pathway;
(c) Predicting whether the patient will be responsive to the inhibitor of the classical/lectin pathway or alternative pathway based on the result of step (b).

13. *In vitro* method for determining the disorder course of a patient with a disorder associated with the complement system, comprising the following steps:

(a) Providing samples of a subject of at least two time points of the disease course;
(b) Detecting the presence of C3/C5 convertase complex in the sample of the subject comprising the steps:

(i) Determining the amount of C4b/C2b complexes in the sample and
(ii) Determining the amount of C3b/Bb complexes in the sample,
wherein a decreased amount of C4b/C2b complexes in the sample of the later time point compared to the sample of the earlier time point indicates an amelioration of the disorder of the classical/lectin pathway and wherein an increased amount of C4b/C2b complexes in the sample of the later time point compared to the sample of the earlier time point indicates worsening of the disorder of the classical/lectin pathway;
and wherein a decreased amount of C3b/Bb complexes in the sample of the later time point compared to the sample of the earlier time point indicates an amelioration of the disorder of the alternative pathway and/or an increased amount of C3b/Bb complexes in the sample of the later time point compared to the sample of the earlier time point indicates worsening of the disorder of the alternative pathway;

(c) Determining the disorder course based on the result of step (b).

**14.** *In vitro* method for determining whether a candidate compound is suitable for the treatment of a disorder of the complement system, wherein the prediction of a suitable candidate compound is based on step (c), comprising the following steps:

(a) Incubating a sample of a subject with a candidate compound;
(b) Detecting the presence of C3/C5 convertase complex in the sample of step (a) comprising the steps:

(i) Determining the amount of C4b/C2b complexes in the sample and
(ii) Determining the amount of C3b/Bb complexes in the sample,

wherein, a decreased amount of C4b/C2b complexes compared to a control compound indicates that the candidate compound is suitable for the treatment of the disorder of the complement system and wherein a decreased amount of C3b/Bb complexes compared to a control compound indicates that the candidate compound is suitable for the treatment of the disorder of the complement system;
(c) Predicting whether a candidate compound is suitable for the treatment of a disorder of the complement system based on the result of step (b).

**15.** Kit comprising:

a) probes for detecting the complexes of C4b and C2b comprising:

- a probe containing an analyte binding moiety specifically binding C4b and a first signal generating moiety, and
- a probe containing an analyte binding moiety specifically binding C2b and a second signal generating moiety, and

b) probes for detecting complexes of C3b and Bb comprising:

- a probe containing an analyte binding moiety specifically binding C3b and a first signal generating moiety, and
- a probe containing an analyte binding moiety specifically binding Bb and a second signal generating moiety.

**16.** Kit according to claim 15, further comprising

c) probes for detecting the complexes of Ig and C1q comprising:

- a probe containing an analyte binding moiety specifically binding Ig and a first signal generating moiety, and
- a probe containing an analyte binding moiety specifically binding C1q and a second signal generating moiety.

and/or
d) probes for detecting the complexes of MASP1/3 and/or MASP2 and MBL comprising:

- a probe containing an analyte binding moiety specifically binding MASP1/3 or MASP2 and a first signal generating moiety, and

- a probe containing an analyte binding moiety specifically binding MBL and a second signal generating moiety.

## Patentansprüche

1. In-vitro-Verfahren zur Klassifizierung einer mit dem Komplementsystem assoziierten Störung, umfassend die folgenden Schritte:

   (a) Bereitstellen einer Probe eines Subjekts;
   (b) Nachweisen des Vorhandenseins des C3/C5-Konvertase-Komplexes in der Probe des Subjekts, umfassend die folgenden Schritte:

   (i) Bestimmen der Menge an C4b/C2b-Komplexen in der Probe und
   (ii) Bestimmen der Menge an C3b/Bb-Komplexen in der Probe,

   wobei eine erhöhte Menge an C4b/C2b-Komplexen im Vergleich zu einer Kontrolle oder einem vorbestimmten Schwellenwert auf eine Störung hinweist, die mit dem klassischen/Lektin-Weg assoziiert ist, und wobei eine erhöhte Menge an C3b/Bb-Komplexen im Vergleich zu einer Kontrolle oder einem vorbestimmten Schwellenwert auf eine Störung hinweist, die mit dem alternativen Weg assoziiert ist;
   (c) Klassifizieren der mit dem Komplementsystem assoziierten Störung auf der Grundlage des Ergebnisses von Schritt (b).

2. In-vitro-Verfahren nach Anspruch 1, wobei die erhöhte Menge an C4b/C2b-Komplexen im Vergleich zu einem vorbestimmten Schwellenwert auf eine mit dem klassischen/Lektin-Weg assoziierte Störung hinweist.

3. In-vitro-Verfahren nach einem der vorhergehenden Ansprüche, wobei in Schritt (b) der Nachweis der Menge des C3/C5-Konvertase-Komplexes durch In-situ-Proximity-Ligation in der Probe bestimmt wird.

4. In vitro-Verfahren nach einem der vorhergehenden Ansprüche, wobei Schritt b) ferner die folgenden Schritte umfasst:

   (i) Bestimmen der Menge des Ig/C1q-Komplexes in der Probe; und/oder
   (ii) Bestimmen der Menge des MASP1/3/MBL-Komplexes und/oder MASP2/MBL-Komplexes in der Probe.

5. In-vitro-Verfahren nach einem der vorhergehenden Ansprüche, wobei die Probe aus der Gruppe ausgewählt ist, die aus einer Körperflüssigkeits- oder Gewebeprobe besteht, wobei die Probe vorzugsweise eine Nierengewebeprobe ist.

6. In-vitro-Verfahren nach einem der vorhergehenden Ansprüche, wobei die Probe eine Plasmaprobe ist.

7. In-vitro-Verfahren nach einem der vorhergehenden Ansprüche, wobei die mit dem Komplementsystem assoziierte Störung aus der Gruppe ausgewählt ist, die aus Nierenerkrankung, Krebs, Autoimmunität, Infektion, Degeneration und Neuropathie besteht.

8. In-vitro-Verfahren nach Anspruch 7, wobei die Nierenerkrankung ausgewählt ist aus der Gruppe bestehend aus systemischem Lupus erythematodes (SLE), thrombotischer Mikroangiopathie, verursacht durch das atypische hämolytischurämische Syndrom (aHUS), dem durch Shiga-Toxin produzierendes *E. coli* verursachten hämolytisch-urämischen Syndrom (STEC-HUS), dem sekundären hämolytisch-urämischen Syndrom (sekundäres HUS), der C3-Glomerulopathie, der Kryoglobulinämie, der C3-Glomerulonephritis, der Dense Deposit Disease, der post-/parainfektiösen Glomerulonephritis, der IgA-Nephropathie, der Kryoglobulinämie und der membranoproliferativen Glomerulonephritis (MPGN).

9. In-vitro-Verfahren nach einem der vorhergehenden Ansprüche, wobei die mit einer Aberration des klassischen/-Lektin-Wegs assoziierte Störung aus der Gruppe ausgewählt ist, die aus Kryoglobulinämie, membranöser Nephropathie und SLE, vorzugsweise SLE, besteht, und wobei die mit einer Aberration des alternativen Wegs assoziierte Störung ein HUS, eine post-/parainfektiöse Glomerulonephritis und C3-Glomerulopathie ist.

10. In-vitro-Verfahren nach einem der vorhergehenden Ansprüche, wobei die Probe aus der Gruppe ausgewählt ist, die aus einer Körperflüssigkeits- oder Gewebeprobe besteht.

**11.** In-vitro-Verfahren nach einem der vorhergehenden Ansprüche, wobei die Gewebeprobe eine Nierengewebeprobe ist.

**12.** In-vitro-Verfahren zur Bestimmung, ob ein Patient mit einer mit dem Komplementsystem assoziierten Störung auf einen Modulator des klassischen/Lektin-Wegs oder des alternativen Wegs ansprechen wird, umfassend die folgenden Schritte:

(a) Bereitstellen einer Probe des Patienten;
(b) Nachweisen des Vorhandenseins des C3/C5-Konvertase-Komplexes in der Probe des Patienten, umfassend die Schritte:

(i) Bestimmen der Menge an C4b/C2b-Komplexen in der Probe und
(ii) Bestimmen der Menge an C3b/Bb-Komplexen in der Probe,

wobei eine erhöhte Menge an C4b/C2b-Komplexen anzeigt, dass der Patient auf einen Inhibitor des klassischen/Lektin-Wegs ansprechen wird und/oder wobei eine erhöhte Menge an C3b/Bb-Komplexen anzeigt, dass der Patient auf einen Inhibitor des alternativen Wegs ansprechen wird;
(c) Vorhersagen, ob der Patient auf den Inhibitor des klassischen/Lektin-Wegs oder des alternativen Wegs ansprechen wird, auf der Grundlage des Ergebnisses von Schritt (b).

**13.** In-vitro-Verfahren zur Bestimmung des Störungsverlaufs eines Patienten mit einer mit dem Komplementsystem assoziierten Störung, umfassend die folgenden Schritte:

(a) Bereitstellen von Proben eines Subjekts zu mindestens zwei Zeitpunkten des Störungsverlaufs;
(b) Nachweisen des Vorhandenseins des C3/C5-Konvertase-Komplexes in der Probe des Subjekts, umfassend die Schritte:

(i) Bestimmen der Menge an C4b/C2b-Komplexen in der Probe und
(ii) Bestimmen der Menge an C3b/Bb-Komplexen in der Probe,
wobei eine verringerte Menge an C4b/C2b-Komplexen in der Probe des späteren Zeitpunkts im Vergleich zu der Probe des früheren Zeitpunkts eine Verbesserung der Störung des klassischen/Lektin-Wegs anzeigt und wobei eine erhöhte Menge an C4b/C2b-Komplexen in der Probe des späteren Zeitpunkts im Vergleich zu der Probe des früheren Zeitpunkts eine Verschlechterung der Störung des klassischen/Lektin-Wegs anzeigt; und wobei eine verringerte Menge an C3b/Bb-Komplexen in der Probe des späteren Zeitpunkts im Vergleich zu der Probe des früheren Zeitpunkts eine Verbesserung der Störung des alternativen Wegs anzeigt und/oder eine erhöhte Menge an C3b/Bb-Komplexen in der Probe des späteren Zeitpunkts im Vergleich zu der Probe des früheren Zeitpunkts eine Verschlechterung der Störung des alternativen Wegs anzeigt;

(c) Bestimmen des Störungsverlaufs auf der Grundlage des Ergebnisses von Schritt (b).

**14.** In-vitro-Verfahren zur Bestimmung, ob eine Kandidatenverbindung für die Behandlung einer Störung des Komplementsystems geeignet ist, wobei die Vorhersage einer geeigneten Kandidatenverbindung auf Schritt (c) basiert, umfassend die folgenden Schritte:

(a) Inkubieren einer Probe eines Subjekts mit einer Kandidatenverbindung;
(b) Nachweisen des Vorhandenseins des C3/C5-Konvertase-Komplexes in der Probe von Schritt (a), umfassend die Schritte:

(i) Bestimmen der Menge an C4b/C2b-Komplexen in der Probe und
(ii) Bestimmen der Menge an C3b/Bb-Komplexen in der Probe,

wobei eine verringerte Menge an C4b/C2b-Komplexen im Vergleich zu einer Kontrollverbindung anzeigt, dass die Kandidatenverbindung für die Behandlung der Störung des Komplementsystems geeignet ist, und wobei eine verringerte Menge an C3b/Bb-Komplexen im Vergleich zu einer Kontrollverbindung anzeigt, dass die Kandidatenverbindung für die Behandlung der Störung des Komplementsystems geeignet ist;
(c) Vorhersagen, ob eine Kandidatenverbindung für die Behandlung einer Störung des Komplementsystems geeignet ist, auf der Grundlage des Ergebnisses von Schritt (b).

**15.** Kit, umfassend:

a) Sonden zum Nachweisen der Komplexe von C4b und C2b, umfassend:

- eine Sonde, enthaltend eine Analytbindungseinheit, die spezifisch C4b bindet, und eine erste signalerzeugende Einheit, und
- eine Sonde, enthaltend eine Analytbindungseinheit, die spezifisch C2b bindet, und eine zweite signalerzeugende Einheit, und

b) Sonden zum Nachweisen von Komplexen aus C3b und Bb, umfassend:

- eine Sonde, enthaltend eine Analytbindungseinheit, die spezifisch C3b bindet, und eine erste signalerzeugende Einheit, und
- eine Sonde, enthaltend eine Analytbindungseinheit, die spezifisch Bb bindet, und eine zweite signalerzeugende Einheit.

**16.** Kit nach Anspruch 15, ferner umfassend

c) Sonden zum Nachweisen der Komplexe von Ig und C1q, umfassend:

- eine Sonde, enthaltend eine Analytbindungseinheit, die spezifisch Ig bindet, und eine erste signalerzeugende Einheit, und
- eine Sonde, enthaltend eine Analytbindungseinheit, die spezifisch C1q bindet, und eine zweite signalerzeugende Einheit,

und/oder
d) Sonden zum Nachweisen der Komplexe von MASP1/3 und/oder MASP2 und MBL, umfassend:

- eine Sonde, enthaltend eine Analytbindungseinheit, die spezifisch MASP1/3 oder MASP2 bindet, und eine erste signalerzeugende Einheit, und
- eine Sonde, enthaltend eine Analytbindungseinheit, die spezifisch MBL bindet, und eine zweite signalerzeugende Einheit.

## Revendications

**1.** Procédé in vitro pour classer un trouble associé au système du complément, comprenant les étapes suivantes:

(a) fourniture d'un échantillon d'un sujet;
(b) détection de la présence de complexe C3/C5 convertase dans l'échantillon du sujet comprenant les étapes suivantes:

(i) détermination de la quantité de complexes C4b/C2b dans l'échantillon et
(ii) détermination de la quantité de complexes C3b/Bb dans l'échantillon,

dans lequel une quantité accrue de complexes C4b/C2b par comparaison à un seuil de contrôle ou prédéterminé indique un trouble associé à la voie classique/des lectines, et dans lequel une quantité accrue de complexes C3b/Bb par comparaison à un seuil de contrôle ou prédéterminé indique un trouble associé à la voie alterne;
(c) classification du trouble associé au système du complément d'après le résultat de l'étape (b).

**2.** Procédé in vitro selon la revendication 1, dans lequel la quantité accrue de complexes C4b/C2b par comparaison à un seuil prédéterminé indique un trouble associé à la voie classique/des lectines.

**3.** Procédé in vitro selon l'une quelconque des revendications précédentes, dans lequel dans l'étape (b) la détection de la quantité du complexe C3/C5 convertase est déterminée par ligature de proximité in situ dans l'échantillon.

**4.** Procédé in vitro selon l'une quelconque des revendications précédentes, dans lequel dans l'étape b) comprend en outre les étapes suivantes :

(i) détermination de la quantité de complexe Ig/C1q dans l'échantillon; et/ou
(ii) détermination de la quantité de complexe MASP1/3/MBL et/ou de complexe MASP2/MBL dans l'échantillon.

**5.** Procédé in vitro selon l'une quelconque des revendications précédentes, dans lequel l'échantillon est choisi dans le groupe constitué de l'échantillon de fluide corporel ou de tissu cellulaire, dans lequel l'échantillon est de préférence un échantillon de tissu rénal.

**6.** Procédé in vitro selon l'une quelconque des revendications précédentes, dans lequel l'échantillon est un échantillon de plasma.

**7.** Procédé in vitro selon l'une quelconque des revendications précédentes, dans lequel le trouble associé au système du complément est choisi dans le groupe constitué de la maladie rénale, du cancer, de l'auto-immunité, de l'infection, de la dégénérescence et de la neuropathie.

**8.** Procédé in vitro selon la revendication 7, dans lequel la néphropathie est choisie dans le groupe constitué du lupus érythémateux systémique (LES), de la microangiopathie thrombotique causée par le syndrome hémolytique et urémique atypique (SHUa), du syndrome hémolytique et urémique à E. coli producteur de shigatoxines (SHU-STEC), le syndrome hémolytique et urémique secondaire (SHU secondaire), la glomérulopathie à dépôts de C3, la cryoglobulinémie, la glomérulonéphrite à dépôts de C3, la maladie des dépôts denses, la glomérulonéphrite post-/para-infectieuse, la néphropathie à IgA, la cryoglobulinémie et la glomérulonéphrite membranoproliférative (GNMP).

**9.** Procédé in vitro selon l'une quelconque des revendications précédentes, dans lequel le trouble associé à une aberration de la voie classique/des lactines est choisi dans le groupe constitué de la cryoglobulinémie, de la néphropathie membranaire et du LES, de préférence le LES et dans lequel le trouble associé à une aberration de la voie alterne est un SHU, une glomérulonéphrite post-/para-infectieuse et une glomérulopathie à dépôts de C3.

**10.** Procédé in vitro selon l'une quelconque des revendications précédentes, dans lequel l'échantillon est choisi dans le groupe constitué de l'échantillon de fluide corporel ou de tissu cellulaire.

**11.** Procédé in vitro selon l'une quelconque des revendications précédentes, dans lequel l'échantillon de tissu est un échantillon de tissu rénal.

**12.** Procédé in vitro pour déterminer si un patient ayant un trouble associé au système du complément sera sensible à un modulateur de la voie classique/des lactines ou de la voie alterne, comprenant les étapes suivantes :

(a) fourniture d'un échantillon du patient ;
(b) détection de la présence de complexe C3/C5 convertase dans l'échantillon du patient comprenant les étapes :

(i) détermination de la quantité de complexes C4b/C2b dans l'échantillon et
(ii) détermination de la quantité de complexes C3b/Bb dans l'échantillon,

dans lequel une quantité accrue de complexes C4b/C2b indique que le patient sera sensible à un inhibiteur de la voie classique/des lectines et/ou dans lequel une quantité accrue de complexes C3b/Bb indique que le patient sera sensible à un inhibiteur de la voie alterne;
(c) prédiction du fait que le patient sera sensible à l'inhibiteur de la voie classique/des lectines ou de la voie alterne d'après le résultat de l'étape (b).

**13.** Procédé in vitro pour déterminer l'évolution du trouble d'un patient ayant un trouble associé au système du complément, comprenant les étapes suivantes:

(a) fourniture d'échantillons d'un sujet d'au moins deux points temporels de l'évolution de la maladie ;
(b) détection de la présence de complexe C3/C5 convertase dans l'échantillon du sujet comprenant les étapes :

(i) détermination de la quantité de complexes C4b/C2b dans l'échantillon et

(ii) détermination de la quantité de complexes C3b/Bb dans l'échantillon,
dans lequel une quantité réduite de complexes C4b/C2b dans l'échantillon du dernier point temporel par comparaison avec l'échantillon du premier point temporel indique une amélioration du trouble associé à la voie classique/des lectines et dans lequel une quantité accrue de complexes C4b/C2b dans l'échantillon du dernier point temporel par comparaison avec l'échantillon du premier point temporel indique une aggravation du trouble associé à la voie classique/des lectines;
et dans lequel une quantité réduite de complexes C3b/Bb dans l'échantillon du dernier point temporel par comparaison avec l'échantillon du premier point temporel indique une amélioration du trouble associé à la voie alterne et dans lequel une quantité accrue de complexes C3b/Bb dans l'échantillon du dernier point temporel par comparaison avec l'échantillon du premier point temporel indique une aggravation du trouble associé à la voie alterne;

(c) détermination de l'évolution du trouble d'après le résultat de l'étape (b).

**14.** Procédé in vitro pour déterminer si un composé candidat est approprié au traitement d'un trouble du système du complément, dans lequel la prédiction d'un composé candidat approprié repose sur l'étape (c), comprenant les étapes suivantes:

(a) incubation d'un échantillon d'un sujet avec un composé candidat;
(b) détection de la présence de complexe C3/C5 convertase dans l'échantillon de l'étape (a) comprenant les étapes:

(i) détermination de la quantité de complexes C4b/C2b dans l'échantillon et
(ii) détermination de la quantité de complexes C3b/Bb dans l'échantillon,

dans lequel une quantité réduite de complexes C4b/C2b par comparaison à un composé de contrôle indique que le composé candidat est approprié au traitement du trouble du système du complément et dans lequel une quantité réduite de complexes C3b/Bb par comparaison avec un composé de contrôle indique que le composé candidat est approprié au traitement du trouble du système de complément;
(c) prédiction du fait qu'un composé candidat est approprié au traitement d'un trouble du système du complément d'après le résultat de l'étape (b).

**15.** Kit comprenant:

a) des sondes pour détecter les complexes de C4b et C2b comprenant:

- une sonde contenant un fragment de liaison à l'analyte se liant spécifiquement à C4b et un fragment générant un premier signal, et
- une sonde contenant un fragment de liaison à l'analyte se liant spécifiquement à C2b et un fragment générant un second signal, et

b) des sondes pour détecter les complexes de C3b et Bb comprenant:

- une sonde contenant un fragment de liaison à l'analyte se liant spécifiquement à C3b et un fragment générant un premier signal, et
- une sonde contenant un fragment de liaison à l'analyte se liant spécifiquement à Bb et un fragment générant un second signal.

**16.** Kit selon la revendication 15, comprenant en outre:

c) des sondes pour détecter les complexes d'Ig et de C1q comprenant :

- une sonde contenant un fragment de liaison à l'analyte se liant spécifiquement à Ig et un fragment générant un premier signal, et
- une sonde contenant un fragment de liaison à l'analyte se liant spécifiquement à C1q et un fragment générant un second signal, et/ou

d) des sondes pour détecter les complexes de MASP1/3 et/ou MASP2 et MBL comprenant :

- une sonde contenant un fragment de liaison à l'analyte se liant spécifiquement à MASP1/3 ou MASP2 et un fragment générant un premier signal, et
- une sonde contenant un fragment de liaison à l'analyte se liant spécifiquement à MBL et un fragment générant un second signal.

# Figure 1

A
Detection Classical/Lectin Pathway:
Proximity Ligation Assay
C4b
C2b
Primary Antibodies
Secondary Antibodies
In Situ PCR
Signals
SLE GN
B
Classical
IgG + C1q
Lectin
MBL+ MASP
C
Alternative
Spontanous initiation
C3
Bb
C3b
C3a
C3 Convertases
C5 Convertases
C5
C5b
C5a
C5b-9: MAC
D
Detection Alternative Pathway:
Proximity Ligation Assay
Primary Antibodies
Secondary Antibodies
In Situ PCR
Signals
aHUS

# Figure 2

A
Classical
convertase
C2+C4b
*
100 µm
B
Alternative
convertase
Factor B+C3b
Δ
100 µm

# Figure 3

A
B
C
D

Figure 4

A
B
C
D
E
F
G
H
I
50 µm
PAS
Classical convertase C4b + C2
Alternative convertase C3b + Factor B

# Figure 5

Preserved glomeruli
Whole biopsy
A
Classical: signals per mm²
B
Alternative: signals per mm²
C
Ratio alternative / classical
D
% alternative convertase
E
Classical: signals per mm²
F
Alternative: signals per mm²
G
Ratio alternative / classical
H
% alternative convertase
SLE
HUS
O-Bx

Figure 6

classical convertase
20000
15000
10000
5000
0
signals per mm²
MGN
PIGN
O-Bx

AP
LP
CP
MBL MASP
IgGC1q
C3bBb
C4bC2b
C3
C5
C5b-9
C8C9

Figure 7

C2bC4b
(Convertase of classical / lectin pathway

Figure 8

AP
LP
CP
Classical pathway:
IgG C1q
14000
12000
10000
8000
6000
4000
2000
0
PLA2R
THSD7A
control

Figure 9A

AP
LP
CP
Lectin pathway:
MBL MASP 1/3
14000
12000
10000
8000
6000
4000
2000
0
-2000
PLA2R
THSD7A
control

Figure 9B

Classical / lectin pathway convertase:
C2bC4b
CP
LP
AP
18000
16000
14000
12000
10000
8000
6000
4000
2000
0
PLA2R
THSD7A
control

Figure 10A

Alternative pathway convertase:
C3bBb
AP
LP
CP
18000
16000
14000
12000
10000
8000
6000
4000
2000
0
-2000
PLA2R
THSD7A
control


Figure 10B

Differences of signal densities
(IgG_C1q) - (MBL_MASP1/3)
19
18
17
16
15
14
13
12
11
10
9
8
7
6
5
4
3
2
1
-20000
-15000
-10000
-5000
0
5000
10000
15000
20000
= THSD7A cases
11-19
= PLA2R cases
1-10

Figure 11A

Differences of signal densities
(IgG_C1q) - (MBL_MASP2)
19
18
17
16
15
14
13
12
11
10
9
8
7
6
5
4
3
2
1
-25000
-20000
-15000
-10000
-5000
0
5000
10000
15000
20000
25000
= THSD7A cases
11-19
= PLA2R cases
1-10

Figure 11B

Differences of signal densities
(C2bC4b) - (C3bBb)
19
18
17
16
15
14
13
12
11
10
9
8
7
6
5
4
3
2
1
-30000
-20000
-10000
0
10000
20000
30000
= THSD7A cases
11-19
= PLA2R cases
1-10

Figure 12

AP
LP
CP
MBL MASP
3 : 6
1 : 9
IgGC1q
C3bBb
1 : 8
2 : 8
C4bC2b
C3
C5
C5b-9
C8C9
= THSD7A cases
= PLA2R cases

Figure 13

Slide scanner
Cover glass
Cell monolayer
Glass slide
Patient serum
Fixation
Convertase and TCC → assembly → detection → quantification

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03068150 A


### Non-patent literature cited in the description


- **JARES-ERIJMAN**. *Nature Biotechnology*, 2003, vol. 21, 1387-1395
- **THURMAN et al.** Complement in kidney disease: core curriculum 2015. *Am J Kidney Dis*, 2015, vol. 65, 156-68
- **ZIPFEL et al.** The role of complement in C3 glomerulopathy. *Mol Immunol*, 2015, vol. 67, 21-30
- **REDDY et al.** Breaking down the complement system: a review and update on novel therapies. *Curr Opin Nephrol Hypertens*, 2017, vol. 26, 123-8
- **RICKLIN D et al.** The renaissance of complement therapeutics. *Nat Rev Nephrol*, 2018, vol. 14, 26-47
- **SÖDERBERG et al.** Direct observation of individual endogenous protein complexes in situ by proximity ligation. *Nat Methods*, 2006, vol. 3, 995-1000
- **VAZQUEZ-LOMBARDI et al.** Challenges and opportunities for non-antibody scaffold drugs. *Drug Discovery Today*, 2015, vol. 20 (10), 1271-1283